# EUROPÄISCHE PATENTSCHRIFT

(11) **EP 1 909 800 B1**
(45) Veröffentlichungstag und Bekanntmachung des Hinweises auf die Patenterteilung: **13.04.2011**
(21) Anmeldenummer: 06762779.4
(22) Anmeldetag: 24.07.2006
(51) Int. Cl.: A61K 31/6615, A61P 35/00

(54) **ACYLGLYCEROPHOSPHOLIPIDE ZUR BEHANDLUNG VON KREBS-BEGLEITERSCHEINUNGEN**
ACYLGLYCEROPHOSPHOLIPIDS FOR TREATING SYMPTOMS CONCOMITANT WITH CANCER
ACYLGLYCEROPHOSPHOLIPIDES POUR TRAITER DES TROUBLES ASSOCIES AU CANCER

(30) Priorität: 22.07.2005 EP 05106747; 17.05.2006 EP 06114119
(43) Veröffentlichungstag der Anmeldung: 16.04.2008
(60) Teilanmeldung: 09179660.7
(73) Patentinhaber: KTB Tumorforschungsgesellschaft mbH, 79106 Freiburg (DE)
(72) Erfinder: MASSING, Ulrich, 79249 Merzhausen (DE)
(74) Vertreter: von Kreisler Selting Werner
(86) Internationale Anmeldenummer: PCT/EP2006/007275
(87) Internationale Veröffentlichungsnummer: WO 2007/009819

(56) Entgegenhaltungen:
- EP-A- 0 179 444
- EP-A- 1 329 219
- WO-A-01/95884
- WO-A-03/099362
- WO-A2-01/82892
- WO-A2-02/36073
- WO-A2-03/068936
- WO-A2-2004/070009
- DE-A- 19 922 193
- US-A- 5 827 836
- US-A1- 2005 129 835

## Beschreibung

Die Erfindung betrifft die Verwendung von Acylglycerophospholipiden, insbesondere von hydrierten Acylglycerophospholipiden und von Phospholipiden mit hohem Omega-3-Fettsäuregehalt, zur Herstellung eines Medikaments zur Therapie von Krebs-Begleiterscheinungen, ausgewählt aus Tumorkachexie, krebsbedingten Verstimmungen und Schmerzen.

### Hintergrund der Erfindung

Phospholipide (nachfolgend kurz "PL") sind eine Hauptkomponente tierischer Zellmembranen. Sie bestehen in der Regel aus einem hydrophilen Kopf, der über eine negativ geladene Phosphatgruppe mit hydrophoben unpolaren Resten verknüpft ist. Die in biologischen Membranen häufigsten PL sind Glycerophospholipide.

Glycerophospholipide (nachfolgend "GPL") sind wie in Formel (I) gezeigt aufgebaut:

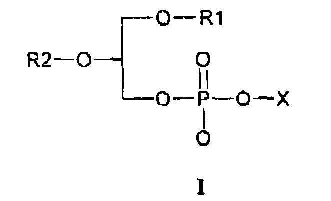

Sie bestehen aus einem hydrophilen Kopf, der über eine negativ geladene Phosphatgruppe in *sn*-3-Position mit einem Glycerinrückgrat und darüber mit einem oder zwei hydrophoben unpolaren Resten R¹ und R² verknüpft ist (IUPAC Compendium of Chemical Technology, 2nd ed. (1997)), Letztere sind in der Regel O-Acylreste aus Fettsäuren mit einer Länge von 14-24 C-Atomen, können jedoch auch *O*-Alkyl- oder *O*-1-Alkenylreste sein. GPL mit einem oder zwei O-Acylresten werden auch als 1-Acyl-, 2-Acyl- bzw. 1,2-Diacylglycerophospholipide bezeichnet oder pauschal als Acylglycerophospholipide (nachfolgend kurz "AGPL") zusammengefasst, Typische Acylglycerophospholipide sind Phosphatidylglycerin, Phosphatidylserin, Phosphatidylethanolamin (nachfolgend "PE"), Phosphatidylinositol (nachfolgend "PI"), Phosphatidsäure (nachfolgend "PA") und Phosphatidylcholin (nachfolgend "PC"). Acylglycerophospholipide sind insbesondere in Lecithin enthalten.

Glycerophospholipide, insbesondere PC oder PC-enthaltende Mischungen wie z. B. Lecithin, finden wegen ihrer Emulgatorwirkung Einsatz als Bestandteil von Nahrungsergänzungsmitteln und Lebensmitteln, in der Kosmetik, in der parenteralen Ernährung und als Hilfsstoffe zur Formulierung von Arzneimitteln. Meist werden dabei nicht hydrierte Phospholipide aus Soja oder Ei verwendet, lediglich zur Formulierung von Medikamenten für die intravenöse Applikation werden hydrierte Phospholipide bevorzugt.

Als eine Alternative zu nativen Phospholipiden in der Tumortherapie wurden vor allem deren Alkylderivate, wie Alkylphosphocholine und Alkylphospholipide, oder Derivate mit anderen nicht-nativen Substituenten vorgeschlagen (US 4,562,005; US 4,775,758; EP 0171968; US 5,489,580; US 6,172,050; WO 01/72289; DE 4408011; Zeisig, R. et al., Anticancer Drug Des. 16(1):19-26 (2001); Arndt, D. et al., Breast Cancer Res. Treat. 43(3):237-46 (1997); DE-A-3304870; US 4,492,659; DE-A-3204735; US 4,565,659; Modolell, M. et al., Cancer Res. 39(11):4681-4686 (1979)). Jedoch verhinderten die ausgeprägten Nebenwirkungen (insbesondere gastrointestinale Nebenwirkungen sowie negative Auswirkungen auf den Blutdruck und die Blutzusammensetzung) die Gabe von zur Tumortherapie ausreichenden Mengen z. B. des vielversprechenden Alkylphosphocholins Hexadecylphosphocholin (Miltefosin) (Verveij, J. et al., J. Cancer reis. Clin. Oncol. 118:606 (1992)), so dass klinische Prüfungen mit der Substanz (orale Applikation) letztlich erfolglos blieben (Verveij, J et al., Eur. J. Cancer 29A:208 (1993); Planting, A.S. et al., Eur. J. Cancer 29A:518 (1993)). Miltefosin wird daher bis heute in onkologischen Indikationen nur als Hautsalbe zur Behandlung von Hautmetastasen des Mammakarzinoms eingesetzt (Miltex^{®}) (Dummer, R. et al., J. Am. Acad. Dermatol. 29:963 (1993)).

Eine Applikation von Miltefosin per Injektion wurde bisher wegen spontaner Hämolyse nicht für eine Anwendung am Menschen in Betracht gezogen. Die biophysikalischen Eigenschaften von Miltefonsin entsprechen denen von Lyso-Phospholipiden, die bei lokal hohen Konzentrationen membranolytische Eigenschaften aufweisen.

Ein Lyso-Phospholipid ist ein durch Phospholipase A-katalysierte Abspaltung oder durch chemische Hydrolyse eines Fettsäurerestes entstehendes, einkettiges Phospholipid mit oberflächenaktiven Eigenschaften, welche rote Blutkörperchen zu lysieren vermögen (daher der Name). Lyso-Phospholipide (1-Acyl- oder 2-Acyl-glycerophospholipide) können aus den verschiedenen zweikettigen, membranbildenden Phospholipiden entstehen. Beispiele für Lyso-Phospholipide sind Lyso-Phosphatidylcholin, Lyso-Phosphatidylethanolamin (Lyso-Kephalin), Lyso-Phosphatidylglycerol, -serin und Lyso-Phosphatidsäure, deren Ausgangs-Phospholipide sämtlich 1,2-Diacylglycerophospholipide sind. Lyso-Phospholipide sind zwar als karzinostatische Verbindungen bekannt (US 4,372,949), haben aber wegen ihrer oberflächenaktiven Eigenschaften bei Applikation der reinen Lyso-Phospholipide in relevanten Dosen ebenso hämolytische Eigenschaften wie die oben_diskutierten Phospholipide, die nicht Acylglycerophospholipide sind (US 4,562,005; US 4,775,758; EP 0171968; US 5,489,580; US 6,172,050; WO 01/72289; DE 4408011; Zeisig, R. et al., Anticancer Drug Des. 16(1):19-26 (2001); Arndt, D. et al., Breast Cancer Res. Treat. 43(3):237-46 (1997); DE-A-3304870; US 4,492,659; DE-A-3204735; US 4,565,659; Modolell, M. et al., Cancer Res. 39(11):4681-4686 (1979)).

Zudem kamen bislang weder Phospholipide noch Lysophospholipide zur Therapie von rumorinduzierter Kachexie oder von weiteren Begleiterscheinungen einer Krebserkrankung wie Fatigue zum Einsatz. Auch eine Verwendung zur Verhinderung oder Reduktion von Metastasierung oder Tumorwachstum ist bislang nicht bekannt.

Glycerophospholipiden, welche üblicherweise zur Liposomenherstellung verwendet werden, wie z. B. Phosphatidylcholin, wird ein eigener antineoplastischer Effekt sogar abgesprochen (DE-A-19959689).

Phospholipide finden Verwendung als Trägerstoffe und Formulierhilfen (Emulgatoren/Vesikeibildner) für Arzneimittel z. B. in Liposomen. Dennoch ist bislang kein antineoplastischer Effekt der Phospholipide selbst beschrieben. Somit beschränkt sich der Einsatz von Acylglycerophospholipiden in der Tumor- bzw. Krebstherapie bislang auf ihren Einsatz als Trägerstoffe zur Formulierung von Wirkstoffen, z. B. in wirkstoffbeladenen Liposomen (WO 99/49716 oder WO 01/95884) oder in einer bizellulären Zusammenstzung (EP-A-0179444). EP-A-1329219 beschreibt zwar die Verwendung von Dimyristoyllecithin in einem Mittel gegen Tumore, dessen Wirkung auf Apoptose und somit der Abtötung bereits existierender Tumorzellen beruht. Eine solche apoptotische Wirkung ist jedoch von antineoplastischen Wirkungen zu unterscheiden, denn letztere bezeichnen gerade nicht die Abtötung von vorhandenen Tumorzellen, sondern die Prophylaxe gegen Neuentstehung von Tumorzellen.

Des weiteren werden Phospholipide zur Formulierung von Zubereitungen zur parenteralen Ernährung verwendet. Derartige Zubereitungen sind Emulsionen und bestehen typischerweise zu 10, 20 oder 30 % aus Triglyceriden (z. B. Sojaöl, MCT (medium chain triglyceride, mittelkettige Triglyceride), Olivenöl, Fischöle oder deren Mischungen (z. B. Soja/Olive 4:1)). Die Emulsionen enthalten des weiteren einen geringen Anteil Phospholipide (Lecithin) aus Hühnerei als Emulgatoren, wobei ein möglichst geringes Phospholipid/Triglycerid-Verhältnis angestrebt wird (z. B. Intralipid, Baxter, 10 % (20 %; 30 %) Emulsion: 10 g (20 g; 30 g) Sojaöl und 0,6 g (1,2 g; 1,2 g) Phospholipid/-100 ml; Lipundin 10 % N, Braun: 8 g Lecithin auf 100 g Sojaöl; Lipofundin MCT 10%, Braun: 8 g Lecithin auf 100g Soja/MCT (1:1)). Emulsionen mit höherem Triglyceridgehalt benötigen zur Emulgation offensichtlich weniger Phospholipid, was als vorteilhaft angesehen wird. Bei niedrigem Phospholipid/Trigycerid-Verhältnis zeigt sich eine bessere metabolische Verträglichkeit mit signifikant geringerer Akkumulation von Phospholipiden und Cholesterol im Plasma (Hartig, W. et al. (Hrsg.), Ernährungs- und Infusionstherapie, 8. Aufl., Thieme (2004)). Die verwendeten Ei-Lecithine sind hochangereicherte Lecithine mit einem Glycerophosphatidylcholin-Anteil von typischerweise 75 oder 80 % (z. B. Lipoid E 75/E 80).

Krebserkrankungen werden in der Regel von einer Reihe von Begleiterscheinungen begleitet, von denen Kachexie, Schmerzempfindungen und Fatigue die bekanntesten sind. Auch die Metastasenbildung, das Wachstum von Rezidivtumoren und das Weiterwachstum eines bereits vorhandenen Tumors (Tumorprogression) sind im weitesten Sinne Krebs-Begleiterscheinungen. Die Linderung und Prophylaxe derartiger Begleiterscheinungen ist das Ziel der Palliativmedizin. Durch die Gabe von Medikamenten ("Palliativa") oder andere therapeutischen oder therapiebegleitende Maßnahmen sollen dabei diese Begleiterscheinungen unterbunden, reduziert oder von vorneherein durch prophylaktischen Maßnahmen verhindert werden.

Als Tumorkachexie bzw. tumorinduzierte Kachexie bezeichnet man ein bei Tumorpatienten häufiges Syndrom mit hochgradiger Gewichtsabnahme und Änderungen der Körperzusammensetzung. Es kommt zum Abbau des Fettgewebes, der Skelettmuskulatur und zur Beeinträchtigung immunologischer Abwehrmechanismen (Tisdale, M.J., Nutrition 17(5):438-442 (2001); Tisdale, M.J., Curr. Opin. Clin. Nutr. Metab. Care 5(4):401-405 (2002)).

De Wys et al. berichteten, dass ein Gewichtsverlust der Tumordiagnose - abhängig von der Tumorentität - in 31-87% aller Fälle vorausgeht (DeWys, W.D. et al., Am. J. Med., 491 (1980)). Ein schwerer Gewichtsverlust von >10% des gesunden Ausgangsgewichts ließ sich bei 15% aller Patienten bei Diagnosestellung feststellen. Tumortherapien sind mit weiterem Appetit- und Gewichtsverlust assoziiert (Costa, G. und Donaldson, S.S., N. Engl. J. Med., 1471 (1979)).

Die tumorinduzierte Kachexie ist ein schwerwiegendes Problem bei der Behandlung vieler Krebspatienten. Längsschnittstudien zeigten, daß Tumorpatienten mit zurückliegenden Gewichtsverlust eine schlechtere Prognose haben als solche mit stabilem Gewicht. Trotz stärkerer unerwünschter Therapiewirkungen ist das Tumoransprechen bei diesen Patienten geringer, ebenso finden sich eine reduzierte Leistungsfähigkeit, eine geringere Bewertung der subjektiven Lebensqualität und ein vermindertes Überleben (DeWys, W.D. et al., Am. J. Med., 491 (1980); Andreyev, H.J.H.,Eur. J. Cancer, 503 (1998); van Eys, J., Cancer Res., 747 (1982)). Neben der Sepsis ist die Kachexie eine häufige direkte Todesursache bei Tumorpatienten; Zahlenangaben schwanken zwischen 5 und 25% (Klastersky, J., Eur. J. Cancer, 149 (1972)).

Pfitzenmaier et al. berichteten, dass 60-70% aller Patienten mit fortgeschrittenem Prostatakarzinom an einer Kachexie leiden (Pfitzenmaier, J. et al., Cancer 97:1211 (2003)).

Die tumorinduzierte Kachexie kann als entzündlicher Prozess gesehen werden, da bei vielen der Patienten neben der Mangelernährung und dem Gewichtsverlust gleichzeitig erhöhte Konzentrationen von Entzündungsmarkern zu beobachten sind (Moldawer, F.F. und Copeland, E.M., Cancer, 1828 (1997)). Die beobachtete Freisetzung von Zytokinen, katabolen Hormonen und weiteren regulatorischen Peptiden scheint zunächst die primäre Reaktion der Wirtsgewebe des Tumorpatienten zu sein (Tisdale, M.J., Science, 2293 (2000)). Zusätzlich können weitere von Tumorzellen freigesetzte Substanzen, wie der "tumor lipid mobilizing factor" (LMF) und der "Proteolyse-induzierende Faktor" (PIF) katabole Signale beitragen und die Zytokinproduktion sowie die Akutphasenreaktion stimulieren (Tisdale, M.J., Science, 2293 (2000)).

Diese systemische Entzündungsreaktion wird heute als Ursache des beobachteten Verlustes von Appetit (Inui, A., Cancer Res., 4493 (1999)) und Körpergewicht (Fearon, K.C.H., World J. Surgery, 584 (1999)) gesehen, die parallel die Tumorprogression fördern kann (Coussens, L.M. und Werb, Z., Nature, 860 (2002)). Zytokin-induzierte metabolische Veränderungen scheinen bei kachektischen Patienten einen Wiederaufbau verlorener Körperzellmasse selbst bei künstlicher Ernährung zu verhindern (Espat, N.J., J. Surg. Oncol. 77, (1995)) und sind mit einer reduzierten Lebenserwartung assoziiert (O'Gormann, P. et, al., Nutrion and Cancer 36 (2000)). Das resultierende Syndrom aus vermindertem Appetit, Gewichtsverlust und Entzündungszustand wird als Kachexie, Tumorkachexie oder Anorexie-Kachexie-Syndrom bezeichnet (cancer anorexia-cachexia syndrome, CACS) (Nelson, K.A., Semin. Oncol., 64 (2000)).

In Deutschland erkranken jährlich ca. 420.000 Personen neu an einer Krebserkrankung (Zahlen Deutsche Krebsgesellschaft (DKG) für das Jahr 2002). Schätzungen der DKG zufolge leiden etwa 70-80 Prozent aller Patienten zumindest zeitweilig an Fatigue (d.h. bei nur einer Fatigue-Periode pro Patient: 315.000 Patienten pro Jahr). Die Ursachen für diese bleierne Müdigkeit und Erschöpfung, die von etwa 10 Tagen bis zu mehreren Monaten andauern kann, sind nicht klar. Diskutiert werden im Wesentlichen die Krebsbehandlung (OP, Bestrahlung, Chemotherapie), weitere Ursachen können aber auch der Einfluss des Tumors auf den Körper sowie seelische Ursachen sein.

Eine spezifische Fatigue-Therapie gibt es bis heute nicht, behandelt wird mit einem Bündel individueller Strategien wie seelische Unterstützung, Training (wenn möglich) oder z.B. eine ausgewogene Ernährung. Eine nebenwirkungsfreie ergänzend bilanzierte Diät, die die Symptome der Fatigue mildern oder beseitigen könnte, wäre bei allen Patienten mit Sicherheit sehr willkommen,

Eine Vielzahl von Studien belegt die positive Wirkung von ω-3-Fettsäuren, u.a. von EPA und DHA, in den Bereichen Atherogenese (Lipid- und Blutdrucksenkung), Reduktion von Herzrhythmusstörungen, Entzündungshemmung, PMS (Prämenstruelles Syndrom) und ADHS (Aufmerksamkeitsdefizit-Hyperaktivitäts-Syndrom). Insbesondere wegen ihrer entzündungshemmenden Wirkung werden Omega-3-Fettsäuren auch als "antientzündliche Fettsäuren" bezeichnet. Besondere Beachtung im Zusammenhang mit AGPL verdienen auch ω-3 Fettsäuren. Sie werden üblicherweise aus Fischölen gewonnen oder Patienten in Form von Fischölen (Triglyceriden) zugeführt. Bei der Einnahme von Omega-3-Fettsäuren in Form von Triglyceriden (Fischöle) erfolgt jedoch nur eine langsame Akkumulation von DHA (Docosahexaensäure) und EPA (Eicosapentaensäure) in den Zielzellen/Zielgeweben und damit ein spätes Einsetzen des angestrebten Effekts (z.B, Verdrängung von Arachidonsäure durch DHA/EPA) und des daraus resultierenden therapeutischen Effektes. Grund hierfür ist die Prozessierung der Triglyceride nach oraler Gabe. Zunächst werden sie nämlich im Magen-Darm-Trakt in freie Fettsäuren und Monoglyceride zersetzt. Diese werden dann von den Darmepithelzellen aufgenommen und anschließend wieder überwiegend in Triglyceride überführt, welche dann zum Aufbau von Chylomikronen verwendet werden. Diese gelangen ins Lymphsystem, dann ins Blut, dann überwiegend in die Leber. Zwischenzeitlich wird ein Teil der Triglyceride hydrolysiert und die dabei freiwerdenden Fettsäuren werden von Gewebszellen und insbesondere von Adipozyten aufgenommen (Einlagerung). In der Leber werden die Chylomikronen abgebaut und in VLDL überführt, die ins Blut abgegeben werden. Diese verlieren im Blut wieder freie Fettsäuren (Triglycerid-Hydrolyse und Aufnahme der Fettsäuren durch Gewebszellen und Adipozyten) und wandeln sich in LDL um. Diese wiederum werden von diversen Geweben aufgenommen (via LDL-Rezeptoren) und in den Zellen abgebaut.

Als Triglycerid applizierte Fettsäuren werden also hauptsächlich zu energetischen Zwecken eingesetzt. Nur ein kleiner Teil dieser Fettsäuren wird zur Biosynthese von Phospholipiden verwendet. So ist z.B. bei Gabe von Fischölen erst nach ca. 6-8 Wochen ein Plateau hinsichtlich eines DHA- und EPA-Einbaus in Lymphozytenmembranen zu beobachten.

Die Bereitstellung einer anderen Applikationsform, welche Omega-3-Fettsäuren schneller verfügbar macht, ist daher eine Aufgabe der vorliegenden Erfindung.

Die der Erfindung zugrundeliegende Aufgabe war es somit, ein Medikament zu entwickeln, das ein Phospholipid als Wirkstoff enthält und zur Therapie von Krebs-Begleiterscheinungen wie der Tumorkachexie und zur Prophylaxe von Metastasenbildung und Tumorwachstum (von Rezidiv- oder Primärtumoren) geeignet ist.

### Kurzbeschreibung der Erfindung

Es wurde nun gefunden, dass Acylglycerophospholipide, insbesondere hydrierte Acylglycerophospholipide (bevorzugt AGPL mit ausschließlich gesättigten Acylresten) und AGPL mit einem hohen Gehalt an Omega-3- oder Omega-9-Acylresten, und vor allem hydriertes PC, antineoplastische und antimetastatische Wirkung haben und tumorinduzierte Kachexie verringern. Dies gilt ganz besonders für AGPLs mit langkettigen Acylresten (Kettenlängen von mehr als 16 C -Atomen).

Zudem wurde gefunden, dass Omega-3-Fettsäuren, verabreicht in Form von Acylglycerophospholipiden, wesentlich schneller und verlustfreier als bei Gabe in Form von Fischölen an ihren Wirkungsort im Körper gebracht werden. Der Wirkungsgrad wird somit erhöht. Der therapeutische Effekt kann sehr viel schneller einsetzen. Anwendungen, in denen bisher eine Supplementierung mit Omega-3-Fettsäuren aufgrund der großen zuzuführenden Mengen (> 2-3 g Fischöl) nur schwer möglich war, werden umsetzbar.

Eine ähnliche Wirkung wie mit AGPL, die reich an Omega-3- oder gesättigen Acylresten sind, kann durch AGPL mit einem hohen Anteil an Omega-9-Acylresteb erzielt werden,

Omega-3-Fettsäurerest-reiche Phospholipide und hydrierte Acylglycerophospholipide verringern bevorzugt tumorinduzierte Kachexie und Fatigue sowie tumorinduzierte Schmerzen, Tumorwachstum und Metastasenbildung.

Die Erfindung betrifft somit
(1) die Verwendung von einem oder mehreren Acylglycerophospholipiden (nachfolgend kurz "AGPL") mit einer Struktur der Formel (I) 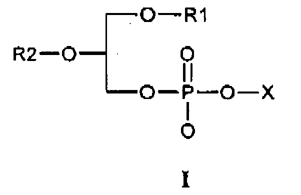 worin
   R¹ und R² unabhängig voneinander ausgewählt sind aus H und C₁₆-₂₄ Acylresten, die geradkettig, verzweigt oder zyklisch, gesättigt oder ungesättigt und mit 1 bis 3 Resten R³ substituiert sein können und in denen eines oder mehrere der C-Atome durch O oder NR⁴ ersetzt sein können;
   X ausgewählt ist aus H, -(CH₂)ₙ-N(R⁴)₃⁺, -(CH₂)ₙ-CH(N(R⁴)₃⁺)-COO⁻ und -(CH₂)ₙ-CH(OH)-CH₂OH, wobei n eine ganze Zahl von 1 bis 5 ist;
   R³ unabhängig vom Auftreten weiterer R³-Reste ausgewählt ist aus H, Niederalkyl, F, Cl, CN und OH; und
   R⁴ unabhängig vom Auftreten weiterer R⁴-Reste ausgewählt ist aus H, CH₃ und CH₂CH₃,
   oder ein pharmakologisch geeignetes Salz desselben als Wirkstoff zur Herstellung eines Medikaments zur Therapie oder Prophylaxe von Krebs-Begleiterscheinungen, ausgewählt aus Tumorkachexie, tumorinduzierten Schmerzzuständen und tumorinduzierter Fatigue;
(2) eine bevorzugte Ausführungsform von (1), wobei das AGPL einen hohen Anteil an gesättigten Acylresten, Omega-3-, und/oder Omega-9-Fettsäureresten besitzt;
(3) eine bevorzugte Ausführungsform von (1) und (2), wobei die AGPL nur gesättigte Acylreste besitzen und insbesondere Phosphatidylcholine mit gesättigten Acylresten sind; und
(4) eine bevorzugte Ausführungsform von (1) bis (3), wobei die AGPL der einzige pharmakologisch aktive Wirkstoff des Medikaments sind.

In einer besonders bevorzugten Ausführungsform von (1) bis (4) ist das Medikament zur Therapie von Tumorkachexie geeignet.

### Kurzbeschreibung der Figuren

Die Erfindung wird anhand der nachfolgenden Figuren in der detaillierten Beschreibung der Erfindung näher erläutert.
- Fig. 1:: Wirkung von Dipalmitoylphosphatidylcholin (DPPC) auf tumorinduzierte Kachexie bei Nacktmäusen, die ein humanes Nierenzellkarzinom (RXF 486) tragen. Punkte: Kontrolle ohne Lipid (n = 3); Kreise: 33 mg DPPC/kg/Tag (Tag 0-4, 7 - 11, 14, 17, 18), orale Applikation (n=3); Rechtecke definieren die Tage, an denen DPPC verabreicht wurde; p: < 0,001.
- Fig. 2:: Wirkung von hydriertem PC auf Tumorprogression bei humanem Weichteilsarkom auf der Nacktmaus. Punkte: Kontrolle ohne Lipid; Kreise: 840 mg EPC-3/kg/Woche (verabreicht an Tag 0, 7 und 14 durch i.v. Applikation in die Schwanzvene; n = 6).
- Fig. 3:: Wirkung von hydriertem PC auf tumorinduzierte Kachexie und Tumor- gewicht in immunkompetenten Mäusen, die ein Nierenzellkarzinom (RENCA-Modell, orthotopes Modell) tragen. Gruppe 1: 50 mg/kg/Tag EPC-3 (n = 5); Gruppe 2: unbehandelte Kontrolle (n = ei). A) Gewicht. Gruppe 1: 84,3 ± 4,3% des Ausgangsgewichtes, Gruppe 2: 76,9 ± 4,2 % des Ausgangsgewichtes. B) Tumorgewicht (p: 0,227).
- Fig. 4:: Lyso-Phosphatidylcholin-Spiegel im Serum von Tumorpatienten mit und ohne Tumorkachexie. Gruppe 1: Tumorpatienten ohne Gewichtsverlust seit Erstdiagnose; Gruppe 2: Tumorpatienten mit weniger als 10 % Ge- wichtsverlust seit Erstdiagnose; Gruppe 3: Tumorpatienten mit mehr als 10 % Gewichtsverlust seit Erstdiagnose (stark kachektisch).
- Fig. 5:: Gewichtsverlauf von Patienten bei Gabe von hydrierten Phospholipiden; vgl. Beispiel 7.
- Fig. 6:: Lysolipid-Abnahme im Medium von Prostata-Karzinom-Zellkulturen (vgl. Bsp. 10). Verwendete Zelllinien: A) LNCaP; B) DU145; C) PC3. Die x-Achse zeigt die Inkubationszeit in h, die y-Achse die prozentuale Abnahme der Lyso-Lipide im Medium.

### Detaillierte Beschreibung der Erfindung

Die erfindungsgemäße Verwendung von AGPL als Wirkstoff in einem Medikament hat sich als eine überraschend wirksame Methode zur Therapie von Krebs-Begleiterscheinungen, insbesondere von tumorinduzierter Kachexie, tumorinduzierter Fatigue, tumorinduzierten Schmerzzuständen, sowie zur Prophylaxe von Metastasenbildung und Tumorwachstum (Tumorprogression) herausgestellt. Das erfindungsgemäße Medikament wird also zur Palliativbehandlung von Krebspatienten eingesetzt. So zeigt die vorliegende Erfindung insbesondere, dass die Gabe von AGPL das Tumorwachstum und die Tumorkachexie verringern kann. Vor allem hydrierte AGPL und Omega-3-Fettsäurerest-reiche AGPL verringern tumorinduzierte Schmerzen, Kachexie und Fatigue sowie Tumorprogression und Metastasenbildung.

Im folgenden werden zunächst einige der verwendeten Begriffe näher definiert:

Ein "Acylglycerophospholipid" ("AGPL,") im Sinne der vorliegenden Erfindung ist ein 1,2-Diacylglycerophospholipid, 1-Acylglycerophospholipid oder 2-Acylglycerophospholipid mit gesättigten oder ungesättigten Acylresten, und schliesst Gemische dieser drei Substanzklassen und deren pharmazeutisch geeigneten Salze ein. So zählen u.a. Phosphatidylcholin, Lyso-Phasphatidylcholin und Lecithin zu den AGPL im Sinne dieser Definition. AGPL besitzt die Struktur der Formel (I) 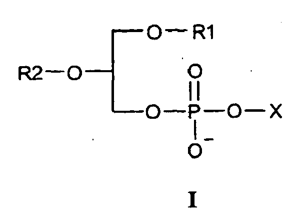 worin
R¹ und R² unabhängig voneinander ausgewählt sind aus H, Alkylcarbonyl-, Alkenylcarbonyl-, Alkinylcarbonyl-, Arylalkylcarbonyl- und Cycloalkylcarbonylresten, worin die Alkylreste geradkettig, verzweigt oder zyklisch, gesättigt oder ungesättigt und mit 1 bis 3 Resten R³ substituiert sein können und in den Alkylresten eines oder mehrere der C-Atome durch O oder NR⁴ ersetzt sein können;
X ausgewählt ist aus H (die Verbindung ist dann eine PA), -(CH₂)ₙ-N(R⁴)₃⁺ (diese Verbindungsklasse umfasst PE und PC), -(CH₂)ₙ-CH(N(F⁴)₃⁺)-COO⁻ (diese Verbindungsklasse umfasst PS) und -(CH₂)ₙ-CH(OH)-CH₂OH (diese Verbindungsklasse umfasst PG), wobei n eine ganze Zahl von 1 bis 5 ist;
R³ unabhängig vom Auftreten weiterer R³-Reste ausgewählt ist aus H, Niederalkyl (worin die Niederalkylreste geradkettig, verzweigt oder zyklisch, gesättigt oder ungesättigt sein können), F, Cl, CN und OH; und
R⁴ unabhängig vom Auftreten weiterer R⁴-Reste ausgewählt ist aus H, CH₃ und CH₂CH₃,
oder ein pharmakologisch geeignetes Salz desselben.

Die Acylreste R¹ und R² sind bevorzugt Alkyjcarbonylreste oder Alkenylcarbonlyreste, besonders bevorzugt Fettsäurereste, wobei geradkettige und unverzweigte Fettsäurereste ganz besonders bevorzugt sind. Die Acylreste können gesättigt oder ungesättigt und gleich oder unterschiedlich lang sein, bevorzugt sind Kettenlängen von C10 bis C24, besonders bevorzugt Kettenlängen von C14 bis C22, ganz besonders bevorzugt von C16 bis C22. Bevorzugt enthalten die erfindungsgemäß verwendeten AGPL Acylreste ausgewählt aus der Gruppe bestehend aus gesättigten Acyresten, Omega-3- und Omega-9-Acylresten sowie Mischungen derselben. Ungesättigte Acylreste sind bevorzugt ausgewählt aus ω-3 und ω-9 Fettsäureresten, insbesondere aus Ölsäure- (18:1), α-Linolensäure- (18:3), Eicosapentaensäure- (20:5; EPA) und Docosahexaensäure-Resten (22:6; DHA). Andererseits sind ungesättigte Acylreste bevorzugt nicht ω-6-Fettsäurereste. Besonders bevorzugt sind bei AGPL, deren Acylreste gesättigt sind, insbesondere solche, die lediglich gesättigte Acylreste mit einer Kettenlänge von C16 oder länger enthalten.

Für den erfindungsgemäßen Einsatz bevorzugt sind AGPL mit natürlich vorkommenden Kopfgruppen, insbesondere Phosphatidylcholine, Phosphatidylethanolamine, Phosphatidylglycerine, Phosphatidylserine und Phosphatidsäuren, ganz besonders AGPL ausgewählt aus der Gruppe der Phosphatidylcholine. Insbesondere Phosphatidylcholine mit hydrierten oder ausschliesslich gesättigten Acylresten sind bevorzugt.

In einer besonders bevorzugten Ausführungsform von (1) werden Verbindungen mit der Struktur der Formel (I) oder deren pharmazeutisch geeignete Salze verwendet, in denen
(i) die Acylreste R¹ und R² unabhängig voneinander 10 bis 24 C-Atome aufweisen, gesättigt sind oder eine oder mehrere Doppelbindungen enthalten, wobei die Zahl der C-Atome bevorzugt ein Vielfaches von 2 ist und die Doppelbindungen nicht konjugiert sind, und wobei die Acylreste besonders bevorzugt Fettsäurereste sind;
(ii) die Niederalkylreste 1-3 C-Atome aufweisen und bevorzugt gesättigt sind; und
(iii) n eine ganze Zahl von 1 bis 3 ist.
Ganz besonders bevorzugt sind Verbindungen der Formel (I), worin
(i) R¹ und R² unabhängig voneinander H oder unverzweigte und unsubstituierte Acylreste sind, welche entweder gesättigt sind (Alkylcarbonylreste) und dann bevorzugt ausgewählt sind aus Lauryl- (n-Dodecanyl-), Myristyl- (n-Tetradecanyl-), Palmitoyl- (n-Hexadecanyl-), Stearyl- (n-Octadecanyl-), Arachinyl- (n-Eicosanyl-), Behenyl- (n-Docosanyl-) und Lignoceryl- (n-Tetracosanyl-)resten und ganz besonders bevorzugt aus Myristyl-, Palmitoyl-, Stearyl- und Arachniylresten, oder ungesättigt sind (Alkenyl- oder Alkinylcarbonylreste) und dann bevorzugt ausgewählt sind aus Omega-3- und Omega-9-Fettsäureresten, besonders bevorzugt aus Oleyl- (18:1), α-Linolenyl-(18:3), Eicosapentaenyl- (20:5) und Docosahexaenyl (22:6)-Resten;
(ii) R³ H ist; und
(iii) X -(CH₂-)ₙ-N(CF₃)₃⁺, -(CH₂-)ₙ-NH₃⁺, oder -CH₂-CH(NH₃⁺)-COO⁻ ist.

Besonders bevorzugt sind AGPL der Formel (II) 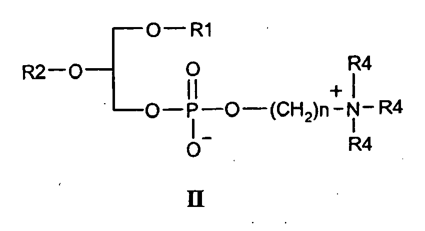 worin
(i) R¹ und R² unabhängig voneinander H oder unverzweigte und unsubstituierte Acylreste sind, welche entweder gesättigt und dann bevorzugt ausgewählt sind aus Palmitoyl-, Stearyl-, Arachinyl-, Behenyl- und Lignocerylresten und ganz besonders bevorzugt aus Palmitoyl-, Stearyl- und Arachniylresten, oder ungesättigt und dann bevorzugt ausgewählt sind aus Oleyl-, α-Linolenyl, Eicosapentaenyl- und Docosahexaenyl-Resten;
(ii) R⁴ CH₃ oder H ist; und
(iii) n 2 oder 3 ist.

Die AGPL im erfindungsgemäßen Medikament und in Therapieverfahren, in welchen dieses Medikament erfindungsgemäß zum Einsatz kommen kann, enthalten bevorzugt eine Mindestmenge an gesättigten, ω-3 oder ω-9 Fettsäureresten oder einer Mischung dieser Fettsäurereste. Diese Mindestmenge definiert sich wie folgt:
(i) bei Diacyl-GPL ist mindestens einer der beiden Acylreste ein gesättigter, ω-3 oder ω-9 Fettsäurerest; oder
(ii) bei AGPL aus natürlichen Quellen, Lyso-AGPL oder Mischungen aus einem oder mehreren AGPL sind mindestens 50 %, bevorzugt mindestens 70 %, besonders bevorzugt mindestens 98% der in den AGPL enthaltenen Acylreste gesättigte, ω-3 und/ oder ω-9 Fettsäurereste. Ganz besonders bevorzugt sind AGPL, welche ausschließlich gesättigte, Omega-3- und/oder Omega-9-Acylreste besitzen.

Von den Diacyl-GPL sind diejenigen bevorzugt, welche zu über 70 %, bevorzugt zu über 98 %, besonders bevorzugt ausschliesslich gesättigte, ω-3 und/ oder ω-9 Acylreste enthalten, die eine Kettenlänge von C16 und/oder C18 haben.

Ist das AGPL dagegen ein Lyso-PL, so ist der Acylrest bevorzugt
(i) ein gesättigter Fettsäurerest mit einer Länge von mindestens C10, bevorzugt mindestens C16, ganz besondes bevorzugt von mindestens C20, oder
(ii) ein ω-3 oder ω-9 Fettsäurerest, der bevorzugt mindestens eine Länge von C16, besonders bevorzugt von C20 hat und insbesondere ein Eicosapentaensäurerest (20:5) oder Docosahexaensäurerest (22:6) ist.

Ganz besonders bevorzugt ist Phosphatidylcholin (PC), das ausschliesslich gesättigte Fettsäurereste, Omega-3- und/oder Omega-9-Fettsäurereste enthält.

Ein erster bevorzugter Aspekt der vorliegenden Erfindung ist die Verwendung von AGPL, welche gesättigte Acylreste enthalten. Dies schliesst auch hydrierte AGPL mit ein, welche durch Hydrierung natürlicher oder synthetischer AGPL hergestellt werden können. Bevorzugt sind bei dieser Verwendung AGPL, welche ausschließlich hydrierte oder gesättigte Acylreste enthalten. Besonders bevorzugt werden hydrierte AGPL verwendet.

Des weiteren bevorzugt sind in diesem Aspekt AGPL, welche PC mit gesättigten Acylresten sind, insbesondere Dipalmitoylphosphatidylcholin (DPPC). DPPC ist unter anderem in Lecithin, vor allem in hydriertem Lecithin enthalten. Unter anderem daher ist auch Lecithin, bevorzugt hydriertes Lecithin, für den erfindungsgemäßen Einsatz geeignet. Ein besonders geeignetes Lecithin ist Ei- oder Soja-Lecithin, vor allem hydriertes Ei- oder Soja-Lecithin.

Ein zweiter bevorzugter Aspekt der vorliegenden Erfindung ist die Verwendung von AGPL, welche Omega-3- und/oder Omega-9-Fettsäurereste enthalten. Besonders bevorzugt sind AGPL mit Omega-3-Fettsäureresten.

Schliesslich ist ein dritter bevorzugter Aspekt der vorliegenden Erfindung die Verwendung von AGPL, welche eine Mischung aus gesättigten, Omega-3- und Omega9-Fettsäureresten enthalten.

Die Herkunft der AGPL (synthetisch oder aus natürlichen Quellen isoliert) ist für ihren erfindungsgemäßen Einsatz irrelevant. Erfindungsgemäße AGPL sind auch Lyso-Acylglycerophospholipide, die sich von AGPL mit zwei Acylresten durch das Fehlen eines Acylrestes in der *sn*-1- oder *sn*-2-Position unterscheiden. Eingesetzt werden können auch handelsübliche Gemische von Glycero phospholipiden oder Fraktionen solcher Gemische. Ein Beispiel ist das sog. Lecithin, das mindestens 20 % Phosphatidylcholin enthalten muss. Solche handelsüblichen Gemische werden dann zur Herstellung der zur Durchführung der Erfindung bevorzugt verwendeten hydrierten PL mit üblichen Methoden hydriert.

"Wirkstoffe" im Sinne der vorliegenden Erfindung sind Verbindungen, die in Lebewesen, insbesondere in Mensch oder Tier, eine physiologische Reaktion hervorrufen können. Sie sind insbesondere in der Therapie eingesetzte Wirkstoffe. Unter einem "pharmakologisch aktiven Wirkstoff' versteht man eine Verbindung, die als Bestandteil eines Arzneimittels die Ursache für dessen Wirksamkeit ist.

Unter "Palliativbehandlung" versteht man eine Therapie mit palliativer Zielsetzung, also in der Krebstherapie die Linderung oder Prophylaxe tumorbedingter Symptome. Zu diesen Symptomen zählen nicht nur tumorbedingte Fatigue, tumorbedingte Schmerzzustände und Tumorkachexie, sondern auch Metastasenbildung und Tumorwachstum. Letztere sollen durch die Pattiativbehandtung gehemmt oder vermindert werden, was in der konventionellen Tumortherapie z.B. durch Palliativbestrahlung geschieht.

"Tumorwachstum" (synonym: Tumorprogression) bezeichnet - wo nicht anders angegeben - das Wachstum jeglichen Tumors, sei er Primärtumor, Rezidivtumor oder Metastase.

Ein "Triglycerid" ist ein Triester des Glycerins mit gleichen oder unterschiedlichen, gesättigten oder ungesättigten Acylresten mit 10 bis 30 C-Atomen. Bevorzugte Triglyceride zur erfindungsgemäßen Verwendung in Kombination mit den AGPL sind Triglyceride enthaltend (bevorzugt ausschließlich) gesättigte Fettsäurereste und diejenigen ungesättigten Fettsäurereste, welche *in vivo* nicht zu Eicosanoiden, insbesondere nicht zu den biologisch hochwirksamen Eicosanoiden der 2er-Serie wie z. B. PGE₂ umgesetzt werden können. Beispiele hierfür sind gesättigte/hydrierte Triglyceride, MCT, Fischöle bzw. Öle aus der Mikroalge Ulkenia (Ärztezeitung vom 26.8.2004), welche beide sehr viel EPA und DHA enthalten, Olivenöle, Rapsöle, Nachtkerzenöle und Lein(samen)öle.

Omega-3-Fettsäuren (synonym: ω-3-Fettsäuren) sind ungesättigte Fettsäuren, deren äußerste Doppelbindung an der - von der Carboxylgruppe gesehen - drittletzten C-C-Bindung liegt. Im Rahmen der vorliegenden Erfindung ganz besonders bevorzugte Omega-3-Fettsäurereste in den AGPL sind alpha-Linolensäure-, EPA- (20:5) und DHA (22:6)-Reste, vor allem bevorzugt sind EPA- und DHA-Reste. Omega-3-Fettsäuren kommen vor allem in Fischen vor, und hier besonders in Kaltwasserfischen. Diese stellen daher im Kontext der vorliegenden Erfindung die bevorzugte natürliche Quelle für Omega-3-Fettsäuren und Omega-3-enthaltende PL dar. Omega-3-Fettsäuren sind essentielle Fettsäuren.

Wie hydrierte Fettsäuren können auch Omega-3-Fettsäuren *in vivo* nicht in hochentzündliche Eicosanoide überführt werden. Sie werden höchstens in weniger entzündungsfördernde Eicosanoide wie z.B. PGE₃ umgewandelt. Deswegen gelten Omega-3-Fettsäuren als antientzündlich und antitumoral. Eine Verschiebung der Fettsäurezusammensetzung einer Zelle, insbesondere der Zellmembran, hin zu erhöhten Omega-3-Fettsäureanteilen kann also Entzündungen entgegenwirken und zur Tumorbekämpfung dienen.

"Marine Phospholipide" (MPL) sind im Kontext der vorliegenden Erfindung aus tierischen Wasserbewohnern gewonnene PL bzw. synthetisch hergestellte PL, deren Zusammensetzung der Zusammensetzung dieser natürlichen PL entspricht. Bevorzugt sind MPL, welche reich an Omega-3-Fettsäureresten sind, also bevorzugt wenigstens 20 Gew.-% Omega-3-Fettsäurereste bezogen auf die Gesamtlipidmenge enthalten. Besonders bevorzugt ist Fischlecithin. Ganz besonders bevorzugt ist MPL aus Fischen, insbesondere aus Fischleber oder Fischrogen, vor allem aus Lachsrogen.

Eine Omega-6-Fettsäure ist eine ungesättigte Fettsäure, deren erste Doppelbindung am sechsten C-Atom gezählt vom Ende der Kohlenstoffkette (wobei die Carboxylgruppe als Anfang gilt) zu finden ist. Prominente Vertreter sind Linolsäure, gamma-Linolensäure und Arachidonsäure.

Eine Omega-9-Fettsäure ist eine ungesättigte Fettsäure, deren erste Doppelbindung am neunten C-Atom gezählt vom Ende der Kohlenstoffkette (wobei die Carboxylgruppe als Anfang gilt) zu finden ist. Omega-9-Fettsäuren können *in vivo* dieselben Wirkungen entfalten wie oben für Omega-3-Fettsäuren beschrieben. Ölsäure ist eine Omega-9-Fettsäure. Olivenöl ist reich an Omega-9-Fettsäure-Triglyceriden, Lecithin ist reich an Oleyl-Rest-enthaltenden AGPL.

Das Medikament in Ausführungsform (1) ist ein Palliativum, da es in der Palliativbehandlung von Krebspatienten zum Einsatz kommt. Zu seiner palliativen Wirkung zählt auch eine antineoplatische Wirkung, es ist also zur Vorbeugung von Tumorwachstum geeignet.

"Krebs-Begleiterscheinungen" im Sinne der vorliegenden Anmeldung umfassen Symptome, welche ein Krebserkrankung begleiten, nämlich tumorinduzierte Kachexie, Schmerzempfindungen und Fatigue. In einer bevorzugten Ausführungsform von (1) besteht die die Gruppe der Krebs-Begleiterscheinungen aus tumorinduzierter Kachexie.

Wichtig im Zusammenhang mit der vorliegenden Erfindung ist, dass insbesondere bei metastasierenden Tumoren, die häufig Kachexie induzieren, oft auch ein erhöhter Spiegel des hauptsächlich mit inflammatorischen Prozessen assoziierten lipolytischen Enzyms sPLA2 zu beobachten ist (Ogawa, M. et al., Res. Comm. Chem. Pathol. Pharmacol. 74(2):241-244 (1991)). Es ist beschrieben, dass die proinflammatorischen Zytokine TNFα, IFNγ, IL-1 und IL-6 die sPLA2 induzieren (Pruzanski, W. et al., Biochim. Biophys. Acta 1403(1):47-56 (1998); Kudo, I. und Murakami, M., Prostaglandins Other Lipid Mediat. 68-69:3-58 (2002)).

Ein erhöhter Spiegel an lipolytischer sPLA2 könnte einen erhöhten zellulären Phospholipidabbau zur Folge haben, der zunächst zur Freisetzung von Lyso-Phospholipiden und freien Fettsäuren in Zellmembranen führen würde. sPLA2 spaltet bevorzugt Fettsäuren aus der 2-Position von Phospholipiden. Häufig ist Arachidonsäure eine der bei der sPLA2-Hydrolyse von Membran-Phospholipiden freigesetzten Fettsäuren.

Der durch eine erhöhte sPLA2-Aktivität verursachte Zellmembranabbau hätte signifikante Folgen für den Tumorpatienten. Da die bei der Spaltung von Phosholipiden freigesetzten Lyso-Phospholipide zu einem Grossteil durch Lysophospholipid-Lipasen weiter abgebaut werden, muß die Zellen neue Phospholipide aufbauen. Dies geschieht zum einen durch *de novo* Synthese (*via* CTP-Phosphocholincytidyltransferase, CTT) oder durch Reacylierung von externem Lyso-PC (aus dem Serum). Letzteres ist wahrscheinlich der Hauptsyntheseweg für neue Phospholipide, da hohe Lyso-PC-Konzentrationen, wie sie im Serum vorkommen (ca. 300 µM) (Raffelt, K. et al., NMR Biomed. 13(1):8-13 (2000); Sullentrop, F. et al., NMR Biomed. 15(1):60-68 (2000)), CTT inhibieren (Boggs, K. P. et al., J. Biol. Chem. 270(13):7757-64 (1995)).

Eine Folge wäre, dass die beim Phosholipidabbau freigesetzten Fettsäuren zu Eicosanoiden umgewandelt werden. Damit hätte eine erhöhte sPLA2-Aktivität eine erhöhte Eicosanoidproduktion der Zellen zur Folge, wobei dieses Eicosanoid bei Tumorzellen oft PGE₂, hergestellt aus Arachidonsäure, ist. PGE₂ wiederum wirkt autokrin auf die Tumorzellen und stimuliert deren Wachstum. Zudem ist ein erhöhter PGE₂-Spiegel mit einer erhöhten Metastasierungsrate assoziiert (Attiga, F. A. et al., Cancer Res. 60(16):4629-4637 (2000)). Eine weitere wichtige Implikation eines erhöhten PGE₂-Spiegels ist zudem die Schmerzsensibilisierung bei Tumorpatienten, da PGE₂ die Erregbarkeit von Nozizeptoren steigert (Brune, K. und Zeilhofer, H.U., Biospektrum 1:36-38 (2004)).

Eine weitere Folge der Freisetzung von Fettsäuren, insbesondere von Arachidonsäure, könnte deren Umsetzung durch das Cytochrom-P450-Enzym CYP2J2 zu den entsprechenden Epoxiden sein. In einer jüngeren Studie (Jiang et al., Cancer Res. 65:4707-4715 (2005)) wurde gezeigt, dass in menschlichen Tumoren die Expression von CYP2J2 hochreguliert wird. CYP2J2 ist eine Epoxygenase, die das Substrat Arachidonsäure in vier verschiedene isomere Epoxyeicosatriensäuren (EET) überführt. EET haben eine apoptosehemmende Wirkung, denn sie schützen Tumorzellen vor der Wirkung von Tumornekrosefaktoren. So erhöhen EET die Lebensdauer der Krebszellen. Überdies fördern sie die Angiogenese (Pozzi, A. et al., J. Biol. Chem. 280:27138-27146 (2005)), die Mitose und die Proliferation von Tumorzellen. Die im Rahmen der vorliegenden Erfindung bevorzugte Applikation von hydrierten AGPL, ? -3-oder ? -9-Fettsäurerest-reichen AGPL führt voraussichtlich zu einer Verminderung des Arachidonsäuregehalts der Tumorzellmembranen, so dass auch weniger tumorfördernde EETs entstehen.

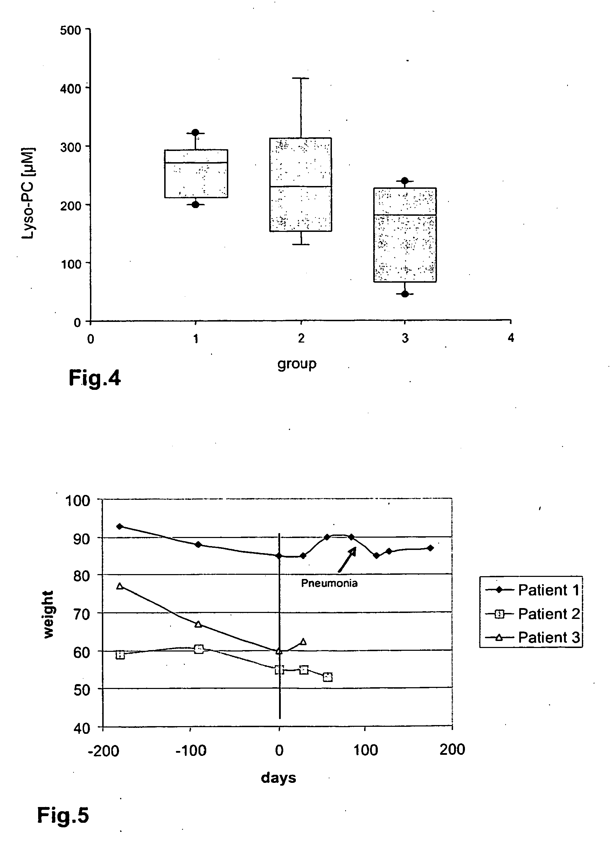

Eine andere weitere Folge des Phospholipidabbaus könnte eine Depletierung von Lyso-PCs im Serum sein, falls diese verstärkt für eine PL-Neusynthese benötigt werden. Eine solche Depletion könnte schädlich für andere Zellen sein, die auch auf Lysophospholipide angewiesen sind wie z. B. die Zellen des Immunsystems. Dies könnte insbesondere ein Problem bei solchen Patienten darstellen, die am cancer anorexia-cachexia syndrome leiden und nicht mehr ausreichend Nahrung für die Wiederherstellung des Lyso-PC-Spiegels aufnehmen können. Ein Hinweis hierauf ist, dass der Lyso-PC-Spiegel bei kachektischen Tumorpatienten sinkt (Gruppe 3 in Bsp. 4, Fig. 4).

Somit ist die erfindungsgemäße Wirkung der AGPL auf das Tumorwachstum, sowie auf die Krebs-Begleiterscheinungen Tumorkachexie, Metastasierung, Fatigue und Schmerzempfinden möglicherweise darin begründet, dass die AGPL den beschleunigten zellulären Phospholipid-Abbau und die damit verbundenen oben beschriebenen Folgen wie die Freisetzung von Arachidonsäure, die Bildung von EET und die Aktivierung von sPLA2 reduzieren.

Die erfindungsgemäße Wirkung des AGPL beruht des weiteren vermutlich darauf, dass es *in vivo* zumindest teilweise in Fettsäuren und Lyso-Phospholipide gespalten wird. Diese beiden Komponenten entfalten dann einzeln (parallel) oder gemeinsam eine Wirkung, welche allein durch die Verabreichung einer der beiden Komponenten nicht problemlos erzielt werden könnte. Im Gegenteil könnte die Gabe von Lyso-AGPL wegen seiner hämolytischen Eigenschaften und der gastrointestinalen Nebenwirkungen sogar schaden, während freie Fettsäuren allein andererseits völlig andere pharmakokinetische und biophysikalische Eigenschaften als AGPL besitzen und daher deren Wirkung im Organismus nicht erzielen könnten.

Eine ganz besonders bevorzugte Ausführungsform von (1) ist die erfindungsgemäße Verwendung des AGPL in Kombination mit einen Triglycerid oder den aus einem Triglycerid herstellbaren freien Fettsäuren, Mono- und Diglyceriden, wobei der Anteil der AGPL in der verwendeten Kombination mindestens 10 Gew.-% aller vorhandenen Lipide beträgt. Hiervon ist die Verwendung des Triglycerids als weiterer Bestandteil des Medikaments bevorzugt. Besagte Verbindungen sind oder enthalten bevorzugt überwiegend oder ausschliesslich hydrierte, ω-3 oder ω-9 Fettsäuren bzw. Fettsäurereste, und sie enthalten nicht oder nur in geringen Anteilen ω-6 Fettsäuren bzw. Fettsäurereste. Mit anderen Worten: sie enthalten oder sind überwiegend oder ausschließlich Fettsäurereste, die *in vivo* nicht zu den biologisch hochaktiven Eicosanoiden umgesetzt werden können. Solche Triglyceriden bzw. die resultierenden Di- und Monogylceride und Fettsäuren sind z. B. gesättigte/hydrierte Triglyceride, MCT, Fischöle bzw. Öle aus der Mikroalge Ulkenia (Ärztezeitung vom 26.8.2004), welche beide sehr viel EPA und DHA enthalten, Olivenöle, Rapsöle, Nachtkerzenöle oder Lein(samen)öle.

Dadurch kann die Wirkung des Medikaments, insbesondere die Wirkung auf die tumorinduzierte Kachexie verstärkt werden. Ursache dieses Effekts ist einerseits die bekannte Wirkung von Triglyceriden als hochenergetisches Lebensmittel und Energielieferant. Andererseits ergibt sich aber aus der gleichzeitigen Anwesenheit der Triglyceride und der AGPL ein zusätzlicher Effekt dadurch, dass die *in vivo* teilweise zu Lyso-PL gespaltenen AGPL durch Umesterung einen Fettsäurerest aus den Triglyceriden bzw. den daraus resultierenden Di- und Mono-Glyceriden oder freien Fettsäuren übernehmen können, wodurch dieser Fettsäurerest nun leichter in Zellmembranen eingebaut und nicht primär der Energieversorgung (Fettsäureoxidation) oder der Einlagerung als Triglycerid in Adipocyten zugeführt wird. Sind die in die Zellmembranen eingebauten Fettsäuren überwiegend hydrierte, ω-3 oder ω-9 Fettsäuren, so können die Zellen möglicherweise weniger PGE₂ herstellen. Dies wiederum kann zu einer Verringerung des Tumorwachstums und der Metastasierung, einer Reduktion des Schmerzempfindens und einer Verbesserung der kachektischen Situation führen.

Ausserdem stellen die Triglyceride ein zusätzliches Reservoir an Fettsäuren zur Verfügung, die nicht zu Eicosanoiden umgesetzt werden können. Dies kann den Teil der Kachexie, welcher durch Eicosanoide verursacht wird, beeinflussen und dadurch die Kachexie reduzieren.

Wesentlich bei der erfindungsgemäßen Verwendung des AGPL in Kombination mit einem oder mehreren Triglyceriden ist, dass der AGPL-Anteil am Gesamt-Upid-Gehalt des Medikaments hoch ist. So sollte dieser AGPL-Anteil mindestens 10 Gew-% betragen, vorzugsweise jedoch mindestens 20 Gew.-%, ganz besonders bevorzugt mindestens 40 Gew-% des Gesamtlipidgewichts. Dies ist gleichbedeutend mit einem Verhältnis AGPL:Triglycerid von 1 : 9, bevorzugt von 2 : 8, besonders bevorzugt von 4 : 6. Grund hierfür ist, dass die AGPL als Wirkstoffe durch die Anwesenheit der Triglyceride in ihrer Wirkung lediglich unterstützt werden. Dies unterscheidet die Medikamente der vorliegenden Erfindung auch von Lipidpräparationen zur künstlichen Ernährung, in denen der PL-Anteil so gering wie möglich gehalten werden muss. Des Weiteren können die AGPL in Kombinantionen mit Triglyceriden ein weites Spektrum von Fettsäureresten (hydriert, ungesättigt) enthalten und aus unterschiedlichsten Quellen stammen (Ei, Soja, Fisch etc.), sie sind also nicht wie die Zubereitungen zur künstlichen Ernährung auf Ei-Lecithin beschränkt.

Eine weitere ganz besonders bevorzugte Ausführungsform ist die erfindungsgemäße Verwendung des AGPL in Kombination mit Substanzen, die eine Wirkung auf die Eicosanoidsynthese haben und so die Wirkung des AGPL synergistisch unterstützen können. Erstens sind dies PLA2-Inhibitoren, insbesondere Inhibitoren der sPLA2 (TypII-sPLA), wie sie z. B. in Uhl et al., Phospholipase A2 Basic and Clinical Aspects in Inflammatory Diseases Vol. 24, Karger, Basel, S. 123-175 (1997) und in DE-A-4234130 beschrieben sind.

Zweitens sind dies Substanzen, die die Cyclooxygenasen 1, 2 oder 3 inhibieren, wie die unspezifischen Verbindungen Acetylsalicylsäure, Paracetamol, Diclofenac, Ibuprofen, Metamizol, Phenazon, Propyphenazon sowie COX-2-Inhibitoren, z. B. Meloxicam (Mobec^{®}), Celecoxib (Celebrex^{®}), Rofecoxib (Vioxx^{®}), Etoricoxib (Arcoxia^{®}), Valdecoxib (Rayzon^{®}), und Parecoxib (Dynastat^{®}).

Diese Substanzen werden in Konzentrationen/Dosen eingesetzt, wie sie für die therapeutische Verwendung der Substanzen, üblich bzw. vom Hersteller vorgeschrieben sind.

Die Dosis der AGPL bei Gabe des erfindungsgemäßen Medikaments beträgt bevorzugt von 2 bis 300 mg/kg und Tag AGPL, besonders bevorzugt von 2 bis 100 mg/kg und Tag. Letzteres gilt vor allem bei Therapie von Tumorkachexie, tumorinduzierten Schmerzzuständen, tumorinduzierter Fatigue und bei Einsatz des Medikaments als Antimetastatikum. Ganz besonders bevorzugt beträgt die Dosis von 2 bis 40 mg/kg und Tag. Dass sich mit dieser Dosis bereits gute Erfolge bei der Therapie von Tumorkachexie, tumorinduzierten Schmerzzuständen und tumorinduzierter Fatigue einstellen könne, zeigt Beispiel 7.

Die Einnahme kann ohne Nebenwirkungen über mehrere Wochen oder sogar Monate erfolgen (vgl. Beispiel 7). Auch eine Gabe über ein oder mehrere Jahre ist wegen der gesundheitlichen Unbedenklichkeit der erfindungsgemäßen AGPL denkbar.

Das erfindungsgemäße Medikament ist zur systemischen Gabe geeignet, besonders zur oralen (p.o.) oder intravenösen (i.v.) Gabe. Die orale Gabe ist bevorzugt, insbesondere falls das Medikament Lyso-AGPL enthält. Die so verabreichten Phospholipide werden im Magen-Darm-Trakt zu Lyso-PL und freie Fettsäuren gespalten. Die freien Fettsäuren werden in den normalen Fettsäurestoffwechsel des Organismus eingeschleust, nicht jedoch die Lyso-PL. Diese werden wahrscheinlich zu einem hohen Anteil direkt in die Blutbahn überführt, wo sie an Albumin binden. Von dort können sie unmittelbar von solchen Zellen aufgenommen werden, die eine Tendenz zur raschen Aufnahme von Lyso-PL besitzen. Hierzu zählen insbesondere Tumorzellen. Der Einbau in die Zellmembran erfolgt dabei in kürzester Zeit.

Die bekannten Nebenwirkungen von Phospholipiden auf das Blutbild bei i.v. Applikation werden bei Einsatz des erfindungsgemäßen Medikaments verhindert, wenn 1,2-Diacylglycerophospholipide verwendet werden. Diese Substanzen sind keine Mizellbildner (Detergenzien), sondern Membranbildner, weswegen sie keine hämolytischen Eigenschaften aufweisen. Daher werden bei intravenöser Anwendung bevorzugt 1,2-Diacylglycerophospholipide verwendet.

Der erfindungsgemäße Anteil des Acylglycerophospholipids an den Gesamtlipiden im Medikament gemäß (1) beträgt mindestens 10 %, bevorzugt mindestens 40 %, ganz besonders bevorzugt 90 - 100 %. Insbesondere kann der AGPL-Anteil 100% betragen, das AGPL ist dann das einzige Lipid im Medikament.

In einer bevorzugten Ausführungsform von (1) ist das AGPL das einzige im Medikament vorhandene Phospholipid, besonders bevorzugt das einzige vorhandene GPL. Im letzteren Fall wiederum bevorzugt ist, dass nur Acylgylcerophosphocholin vorhanden ist.

Das AGPL kann als einziger Wirkstoff im Medikament vorhanden sein, es kann jedoch auch mit anderen Wirkstoffen kombiniert werden. Im letzteren Falle ist das AGPL bevorzugt der einzige Wirkstoff aus der Verbindungsklasse der Lipide, insbesondere aus der Klasse der Phospholipide; Das Acylglycerophospolipid wird in einer besonders bevorzugten Ausführungsform jedoch als einziger im Medikament enthaltener pharmakologisch aktiver Wirkstoff eingesetzt.

Das erfindungsgemäße Medikament kann wie für lipidhaltige Medikamente üblich formuliert sein, z. B. als Liposomen und liposomale Formulierungen, als übliche Emulsion, als Tabletten, Kapseln oder als Pulver zum Einrühren in Nahrungsmittel. In der bevorzugten Formulierung als Tablette kann die Konzentration der AGPL bis zu 100 % betragen. Weiterhin bevorzugt ist die Formulierung als Liposomen, wobei die AGPL ein Teil der Liposomenmembran sind, in welcher aber auch noch Nicht-AGPL eingebaut sein können. Beispiele für derartige Nicht-AGPL sind Cholesterol und negativ oder positiv geladene Amphiphile (z. B. DOTAP).

Die Dosierung des Medikaments wird von dem behandelnden Arzt individuell auf den jeweiligen Patienten angepasst. Sie richtet sich u. a. nach der Art der Krankheit, der Schwere der zu behandelnden Symptome, der konstitutionellen Beschaffenheit des Patienten usw., wobei üblicherweise Dosen von 2-300 mg/kg Körpergewicht/Tag und insbesondere die oben genannten bevorzugten Dosisbereiche in Betracht kommen.

Ein besonderer Vorteil der Ausführungsform (1) liegt in ihrer Bedeutung für die Herstellung von Medikamenten zur Therapie von Tumorkachexie.

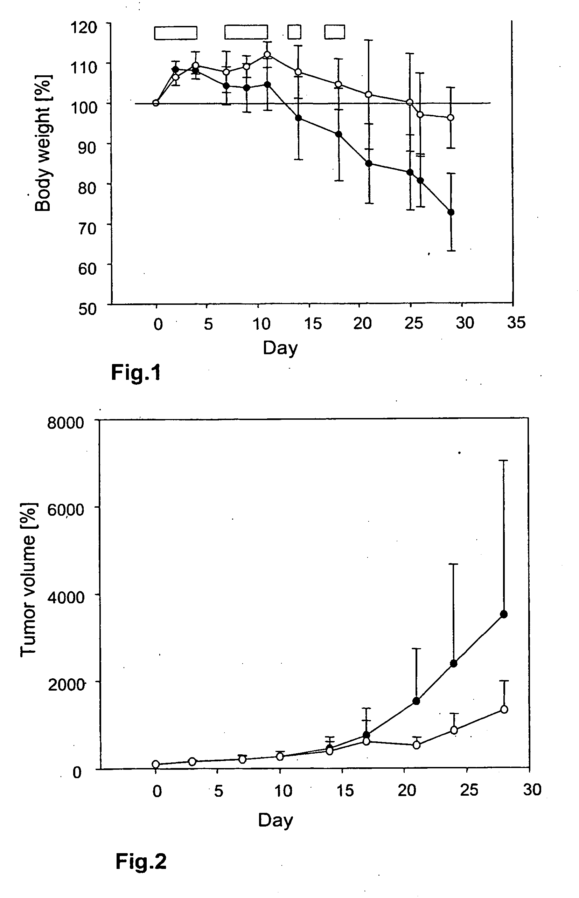

So besitzt Dipalmitoylphosphatidylcholin Wirkung auf tumorinduzierte Kachexie. Gezeigt wird diese Wirkung in Beispiel 1 anhand des Gewichtsverlaufs von Nacktmäusen, die ein humanes Nierenzellkarzinom (RXF 486) tragen. Dieser Tumor wirkt kachexieinduzierend. Durch orale Gabe eines synthetischen PC mit hydrierten Fettsäureresten (Dipalmitoylphosphatidylcholin, DPPC) als liposomale Formulierung in Kochsalzlösung konnte der Gewichtsverlust im Vergleich zu einer unbehandelten Kontrollgruppe praktisch gestoppt werden (Fig. 1). Auch eine positive Wirkung von hydrierten Phospholipiden auf den Gewichtsverlauf bei menschlichen Patienten mit tumorinduzierte Kachexie ist nachweisbar (Bsp. 7).

Die Medikamente der Ausführungsform (1) können auch zur Palliativtherapie bei bestimmten Tumoren in Mensch und Tier, insbesondere zur Vorbeugung oder Reduktion des Wachstums dieser Tumore oder von daraus gebildeten Metastasen (vgl. Beispiel 2, 7 und 8) eingesetzt werden. Bevorzugt sind dies Tumore, für die eine verstärkte sPLA2-Expression beobachtet wurde, also insbesondere Tumore des Gastrointestinaltraktes, des Brustkrebses, des Ovarialkarzinoms, des Pankreaskarzinoms, des Lungenkarzinoms und vor allem des Prostatakarzinoms (Ogawa, M., in: Uhl, W. et al., Phospholipase A2 Basic and Clinical Aspects in Inflammatory Diseases Vol. 24, Karger, Basel, S.200-204 (1997); Yamashita, S. et al., Clin. Chim. Acta 228(2):91-99 (1994); Yamashita, S. et al., J. Cancer 69(6):1166-1170 (1994); Graff, J.R. et al., Clin. Cancer Res. 7(12)3857-3861 (2001)). Weitere und besonders bevorzugte Anwendungsgebiete sind die Palliativtherapie der Begleiterscheinungen einschließlich Tumorwachstums von Weichteilsarkomen und Nierenzellkarzinomen.

Schließlich sind auch solche Tumore bevorzugt, welche *in vitro* einen hohen Lyso-PC-Verbrauch zeigen und damit geeignete Ziele einer Therapie mit hydrierten oder Omega-3- bzw. Omega-9-haltigen AGPL sind. Es sind dies Mammakarzinome, Prostatakarzinome, Pankreas-Karzinome, Glioblastome und leukämische Zellen, vor allem Prostatakarzinome (vgl. Bsp. 10).

So wirkt hydriertes PC auf die Tumorprogression bei einem humanen Weichteilsarkom auf der Nacktmaus. Dieser Tumor wirkt nicht kachexieinduzierend. Durch intravenöse Gabe von hydriertem Lecithin als liposomale Formulierung mit Cholesterol konnte eine Verlangsamung des Tumorwachstums erreicht werden (Beispiel 2; Fig. 2).

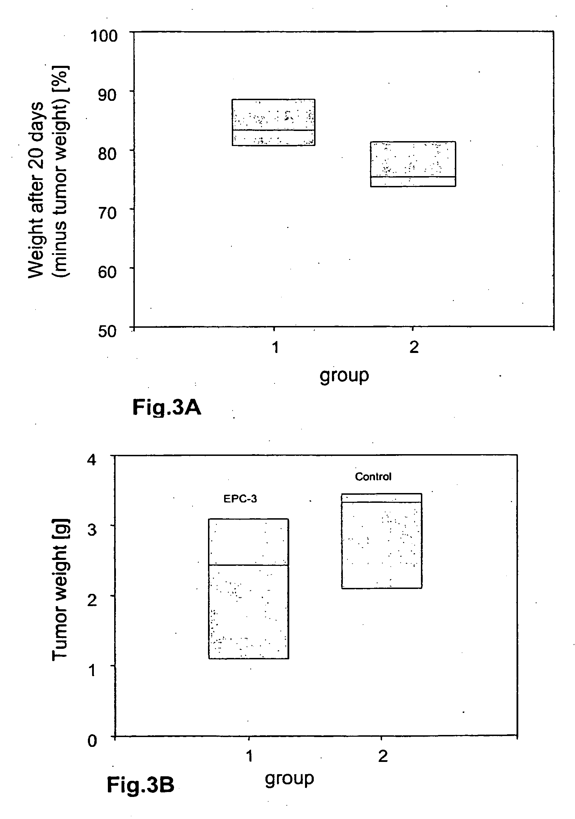

Auch in einem orthotopen Tumormodell ist eine Reduktion der tumorinduzierten Kachexie verbunden mit einer Reduktion des Tumorwachstums durch Gabe von hydriertem Ei-PC nachweisbar (Beispiel 3; Fig. 3).

Dabei ist überraschend, dass die AGPL nicht zur Reduktion des Tumors selbst einsetzbar sind. Denn die Gabe der AGPL an Krebspatienten wirkt nur palliativ, resultiert aber nicht in einer Verkleinerung des Tumors oder Abtötung der Tumorzellen (vgl. Bsp. 2 und 7). Hierin unterscheidet sich die Verwendung der vorliegenden Anmeldung von EP-A-1329219. EP-A-1329219 beschreibt die Verwendung von Dimyristoyllecithin in einem Mittel gegen Krebs, das die Apoptose induziert. Dagegen findet bei der Gabe des erfindungsgemäßen Medikaments keine Apoptose und somit auch keine Abtötung des Tumorgewebes statt. So induzieren Liposomen aus hydriertem Ei-Lecithin (enthaltend vorwiegend C16/C18-Acylreste) keinerlei Zelltod in Tumor-Zellkulturen (vgl. Bsp. 11, in dem Kulturen mit und ohne EPC-3-Zusatz gleichermaßen proliferierten). Bei einem Heilversuch mit drei finalen Patienten durch Gabe von S-75-3N (vgl. Bsp. 7), hydriertem Sojalecithin, das praktisch nur Acylreste mit einer Kettenlänge von mindestens C16 enthält (Analyse siehe Bsp. 9), wurde keine Wirkung auf den Tumor, wohl aber eine Gewichtsstabilisierung sowie Gemütsaufhellung und Schmerzreduktion (bzw. eine Reduktion des Schmerzmittelbedarfs) festgestellt. Des weiteren wurde bei Verabreichung von hydriertem Ei-Lecithin an Mäuse mit Weichteilsarkom keine Reduktion des Tumors festgestellt (Bsp. 2), sondern lediglich eine Verlangsamung des Tumorwachstums.

Auch stellten Nagami et al. (Bioorganic & Medicinal Chemistry Letters 16:782-785 (2006)) fest, dass Dimyristoyl-PC Apoptose und Nekrose in Tumorzellen erzeugt, Di-C16-PC (DPPC) hingegen keinen derartigen Effekt auf Tumorzellen zeigt.

Dies begründet, warum AGPL, deren Acylreste ausschliesslich Kettenlängen von mindestens C16 haben, im Rahmen der vorliegenden Erfindung bevorzugt sind.

Besonders hervorzuheben ist auch noch die Wirkung der erfindungsgemäßen AGPL, insbesondere von hydriertem Phosphatidylcholin, auf die Metastasenbildung (vgl. Bsp. 8). Die Metastasenbildung vor allem in der Leber wird durch Gabe eines erfindungsgemäßen Medikaments deutlich reduziert oder sogar völlig unterbunden.

Ein weiterer Aspekt der Erfindung ist die Reduktion des Schmerzmittelbedarfs bei Krebspatienten, denen das erfindungsgemäße Medikament verabreicht wird. Dieser aussergewöhnliche Effekt zeigte sich z.B. bei Patient 1 in Bsp. 7, bei dem in Anbetracht des fortgeschrittenen Krankheitsstadiums nicht mit einem so aussergewöhnlichen Ergebnis zu rechnen war, insbesondere nicht damit, dass die Schmerzmitteltherapie zeitweise völlig unterlassen werden konnte.

Insgesamt gesehen führt somit die Verabreichung de erfindungsgemäßen Medikaments zu einer Stabilisierung des körperliche und seelischen Befindens des Patienten (keine weitere Verschlechterung, wie sie sonst bei Tumorerkrankungen beobachtet wird), und dies selbst bei Patienten in einem sehr späten oder finalen Stadium der Krebserkrankung (vgl. Bsp. 7).

Werden Omega-3- oder Omega-9-Fettsäurerest-reiche AGPL, wie MPL oder andere Omega-3-Fettsäurerest-reiche AGPL, insbesondere jedoch MPL, zur Durchführung der Erfindung verwendet, so hat die Erfindung mehrere spezielle bevorzugte Aspekte, die im folgenden dargestellt werden:

In einem bevorzugten Aspekt der Verwendung von Omega-3-Fettsäurerestreichen AGPL ist das Medikament zur Therapie von tumorassozüerten Problemen, insbesondere von tumorinduzierter Kachexie, Fatigue, Schmerzzuständen und der Metastasierung geeignet. Ganz besonders gut geeignet ist es zur Behandlung von tumorinduzierter Kachexie.

Werden erfindungsgemäß Omega-3- bzw. Omega-9-Fettsäurereste in Glyceridform verabreicht, so geschieht dies bevorzugt nicht als Triglycerid, sondern als AGPL. Omega-3-Fettsäurerest-reiche AGPL werden bevorzugt als Bestandteil von MPL verabreicht, Omega-9-Fettsäurerest-reiche AGPL als Bestnadteil von Lecithin oder als Dioleylphosphatidylcholin.

Die Omega-3- bzw. Omega-9-Fettsäurerest-reichen AGPL sind bevorzugt ausgewählt aus PC, Lyso-PC, PI und PE, wobei PC besonders bevorzugt sind.

Bevorzugte Omega-3-Fettsäurereste in den Omega-3-Fettsäurerest-reichen AGPL sind langkettige Omega-3-Fettsäurereste, vor allem mit Kettenlängen von mindestens C20, besonders bevorzugt DHA- und EPA-Reste.

Bevorzugte Omega-9-Fettsäurereste in den Omega-9-Fettsäurerest-reichen AGPL sind langkettigte Omega-9-Fettsäurereste, vor allem mit Kettenlängen von mindestens C18, besonders bevorzugt Ölsäure-Reste.

Die Omega-3- und Omega-9-Fettsäurerest-reichen AGPL für die erfindungsgemäße Verwendung werden entweder synthetisch hergestellt oder stammen aus natürlichen Quellen. Letzteres ist bevorzugt. Besonders bevorzugt stammen die Omega-3-Fettsäurerest-reichen AGPL aus marinen Lipidquellen ("marine AGPL", "MPL"), insbesondere aus tierischen Wasserbewohnern wie Fisch, vor allem Kaltwasserfisch. Ganz besonders bevorzugt stammen sie aus Fischleber oder Fischrogen, vor allem Lachsrogen. Die Omega-9-Fettsäurerestreichen AGPL stammen bevorzugt aus Lecithin oder werden im Rahmen der vorliegenden Erfindung in Form von Lecithin verwendet, oder sie sind Dioleylptiosphatidylcholin.

Der Omega-3- bzw. Omega-9-Fettsäurerestanteil in den erfindungsgemäßen Medikamenten und Zusammensetzungen beträgt bevorzugt wenigstens 20 Gew.-%, besonders bevorzugt wenigstens 35 Gew.-%, ganz besonders bevorzugt wenigstens 45 Gew.-% der Gesamt-Lipidmenge. In einer ganz besonders bevorzugten Ausführungsform der Erfindung machen die Omega-3- und/oder Omega-9-Fettsäurereste 50 Gew.-% oder mehr der Gesamt-Lipidmenge im erfindungsgemäßen Medikament bzw. der erfindungsgemäßen Zusammensetzung aus.

Des weiteren beträgt das Gewichtsverhältnis von Omega-3 und/oder Omega-9-zu Omega-6-Fettsäureresten in den verwendeten AGPL vorzugsweise wenigstens 10 : 1, besonders bevorzugt wenigstens 15 : 1 und ganz besonders bevorzugt wenigstens 18 : 1. In einer speziell bevorzugten Ausführungsform, nämlich bei Verwendung von MPL wie in Beispielen 5 bis 6, beträgt es 21 : 1.

Omega-3- bzw. Omega-9-Fettsäurerest-reiche AGPL können im Gemisch mit anderen Substanzen eingesetzt werden, bevorzugt im Gemisch mit Triglyceriden, ganz besonders bevorzugt mit Triglyceriden aus der gleichen natürlichen Quelle (z.B. Lachsrogen). Dabei liegt der Anteil der AGPL in der verwendeten Zusammensetzung bevorzugt bei wenigstens 5 Gew.-%, besonders bevorzugt bei wenigstens 15 Gew.-%, ganz besonders bevorzugt bei wenigstens 30 Gew.-% der Gesamtlipide. Bei dem in den Beispielen 5 bis 6 verwendeten MPL lag der AGPL-Anteil bei 30 Gew.-%, in Lecithin (Omega-9-Fettsäurerest-reich) kann er noch deutlich höher liegen.

Die Omega-3- bzw. Omega-9-Fettsäurerest-reichen Zusammensetzungen werden bevorzugt oral verabreicht. Die Dosis für Patienten mit tumorassoziierten Problemen beträgt bevorzugt wenigstens 150 mg AGPL pro Tag, besonders bevorzugt wenigstens 300 mg/Tag, ganz besonders bevorzugt wenigstens 450 mg/Tag.

Eine Zubereitung und Verwendung der AGPL wie oben definiert, bevorzugt von hydriertem Lecithin und von MPL, kann ebenfalls als Nahrungsergänzungsmittel eingesetzt werden. Ein derartiges Nahrungsergänzungsmittel kann als Ergänzung zur Therapie von tumorassoziierten Problemen wie Kachexie, Fatigue, Schmerzzuständen, und zur Vorbeugung von Metastasierung eingesetzt werden. Eine nebenwirkungsfreie ergänzend bilanzierte Diät, die in der Lage ist, besser als die bisher auf dem Markt befindlichen Omega-3-Produkte (insbesondere Fischöle) oder andere Produkte die Kachexie zu mildern, sollte von einem Großteil der betroffenen Patienten akzeptiert werden und ist daher ebenfalls eine bevorzugter Aspekt dieser Erfindung. Denn selbst das unangenehme Aufstoßen, das oft durch Fischöle hervorgerufen wird, tritt nach ersten Erfahrungen mit MPL nicht mehr auf. Ein solches Produkt kann auch prophylaktisch eingesetzt werden.

Die Erfindung wird anhand der nachfolgenden Beispiele näher erläutert.

### Beispiele

### Beispiel 1: Wirkung von hydriertem PC auf den Gewichtsverlauf von Nacktmäusen die ein humanes Nierenzellkarzinom (RXF 486) tragen.

Tumore aus dem humanen Nierenzellkarzinom RXF 486 wirken stark kachexieinduzierend.

Es wurden weibliche thymusaplasierte BALB/c Nacktmäuse (nu/nu) (Charles River, Frederick, Md.) im Alter von 8 bis 10 Wochen eingesetzt. Die Tiere hatten ein Gewicht von 21-23 g und wurden unter einem natürlichen Tag/Nacht-Zyklus gehalten. Sie erhielten Wasser und Nagerfutter (Altromin, Lage, Deutschland) *ad libitum.*

3-5 mm (Durchmesser) große Fragmente des humanen Nierenzellkarzinoms RXF 486 wurden subkutan zwischen die Vorder- und Hinterflanke der Tiere implantiert. Die Therapieexperimente wurden begonnen, wenn die Tumore eine Größe von 30-40 mm³ erreicht hatten.

Zur Therapie wurde synthetisches PL (Dipalmitoylphosphatidylcholin, DPPC; Sygena, Liestal, Schweiz) eingesetzt. Es wurde in einer Konzentration von 33 mg/ml in 0,9 % (w/v) Kochsalzlösung dispergiert (Ultraschalldispergierung in 50 ml Falcon-Tube), so dass eine heterogene liposomale Dispersion entstand. Für die oral Applikation (p.o.) wurde die Dispersion mit Kochsalzlösung auf 3,3 mg/ml eingestellt.

Die Tiere wurden an den Versuchstagen 0 - 4, 7 - 11, 14, 17 und 18 mit jeweils 33 mg/kg Körpergewicht DPPC p.o. per Schlundsonde behandelt (Applikationsvolumen: 10 ml/kg). Die Kontrolltiere erhielten nur Kochsalzlösung. Das Gewicht der Tiere wurde an den Tagen 0, 2, 4, 7, 9, 11, 14, 18, 21, 25, 26 und 29 bestimmt und ist in Fig. 1 in % des Gewichts des Tages 0 dargestellt.

Ergebnis: Durch orale Gabe des DPPC als liposomale Dispersion in Kochsalzlösung konnte der Gewichtsverlust im Vergleich zu einer unbehandelten Kontrollgruppe praktisch gestoppt werden (Fig. 1).

### Beispiel 2: Wirkung von hydriertem PC auf die Tumorprogression bei einem humanen Weichteilsarkom auf der Nacktmaus.

Tumore aus dem humanen Weichteilsarkom SXF 1301 wirken nicht kachexieinduzierend.

Es wurden weibliche thymusaplasierte BALB/c Nacktmäuse (nu/nu) (Charles River, Frederick, Md.) im Alter von 8 bis 10 Wochen eingesetzt. Die Tiere hatten ein Gewicht von 21 - 23 g und wurden unter einem natürlichen Tag/Nacht-Zyklus gehalten. Sie erhielten Wasser und Nagerfutter (Altromin, Lage, Deutschland) *ad libitum*.

3 - 5 mm (Durchmesser) große Fragmente des humanen Weichteilsarkoms SXF 1301 wurden subkutan zwischen die Vorder- und Hinterflanke der Tiere implantiert. Die Therapieexperimente wurden begonnen, wenn die Tumore eine Größe von 150 - 400 mm³ erreicht hatten.

Hydriertes Ei-Lecithin (Ei-Phosphatidylcholin, Ei-PC; EPC-3 der Fa. Lipoid, Ludwigshafen, Deutschland) wurde als liposomale Formulierung mit Cholesterol (Merck, Darmstadt, Deutschland) in Phosphatpuffer (20 mM Phosphat, 130 mM NaCl, pH 7,4) in einer Dosis von 840 mg/kg wöchentlich über drei Wochen hinweg durch Injektion in die Schwanzvene von 6 Tieren gegeben (Tage: 0, 7, 14). Das molare Verhältnis Lecithin:Cholesterol in der liposomalen Formulierung betrug 55 : 45, die Liposomen hatten eine Größe von 40 - 60 nm. Das Injektionsvolumen war 10 ml/kg, die verabreichte Gesamtlipidmenge betrug 2,17 mmol/kg. Kontrolltiere (n = 6) erhielten an den Tagen 0, 7 und 14 nur 0,9 % (w/v) Kochsalzlösung.

Die Tumorvolumina wurden durch Calipervermessung (Länge x Breite²) der subkutanen (tastbaren und vermessbaren) Tumore an den Tagen 0, 3, 7, 10, 14, 17, 21, 24 und 28 bestimmt.

Ergebnis: Es konnte durch Gabe des hydrierten Lecithins in der Dosis von 840 mg/kg/Woche i. v. eine Verlangsamung des Tumorwachstum im Vergleich zur Kontrollgruppe um den Faktor 2 erreicht werden. Das Ergebnis ist in Fig. 2 dargestellt.

### Beispiel 3: Wirkung von hydriertem PC auf den Gewichtsverlauf und das Tumorvolumen bei immunkompetenten Mäusen, die ein Nierenzellkarzinom (RENCA-Modell, orthotopes Modell) tragen.

Der RENCA-Tumor wirkt stark kachexieinduzierend.

Es wurden weibliche BALB/c-Mäuse (Charles River, Frederick, Md.) im Alter von 6 bis 8 Wochen eingesetzt. Die Tiere hatten ein Gewicht von ca. 20 g und wurden unter einem natürlichen Tag/Nacht-Zyklus gehalten. Sie erhielten Wasser und Nagerfutter (Altromin, Lage, Deutschland) *ad libitum.*

RENCA-Tumorzellen (murines Nierenzellkarzinom) wurden bei den Tieren operativ durch Injektion in die Niere (genauer: in den subcapsularen Spalt in der linken Niere) eingebracht. Eine Woche nach Instillation von 10⁶ RENCA-Tumorzellen war der Tumor bereits makroskopisch sichtbar und es wurde mit der Behandlung der Mäuse begonnen (Tag 1). Die Tiere hatten zu diesem Zeitpunkt ein Gewicht von 16 - 23 g.

Zur Therapie wurde hydriertes Ei-Lecithin (Ei-Phosphatidytchotin, EPC-3; Fa. Lipoid, Ludwigshafen) eingesetzt. Es wurde in physiologischer Kochsalzlösung durch Ultraschall bei ca. 50 °C dispergiert. Es entstand eine heterogene liposomale Dispersion, die mit Ringerlösung auf 5 mgEPC-3/ml eingestellt wurde.

Den Tieren wurden 10 ml/kg der EPC-3-Dispersion (entsprechend 50 mg/kg EPC-3) per Schlundsonde p. o. an den Tagen 1 - 5, 8 - 12, 15 - 20 verabreicht. Die Kontrollgruppe wurde nichts verabreicht.

Das Gewicht der Tiere wurde an den Tagen 1, 3, 6, 8, 10, 13, 15, 17 und 20 gemessen. Das Tumorgewicht wurde am Tag 20 nach Tötung der Tiere durch Wiegen der Niere bestimmt. Da es sich um ein orthotopes Tumormodell handelt und Ort des Tumors die Niere war, wurde diese Messung am Ende des Experimentes vorgenommen. Das Körpergewicht an Tag 20 wurde um das Tumorgewicht korrigiert und ist in Fig. 3A wiedergegeben.

Ergebnis: Durch orale Gabe von 50 mg/kg/Tag hydriertem Ei-PC als liposomale Formulierung in Ringerlösung konnte der Gewichtsverlust im Vergleich zu einer unbehandelten Kontrollgruppe signifikant vermindert werden (p: > 0,017; Fig. 3A).

Des weitere resultierte die EPC-3-Behandlung der RENCA-Mäuse nach 20 Tagen in einem Trend zu geringerem Tumorgewicht (p: 0,227; Fig. 3B).

### Beispiel 4: Bestimmung des Lyso-Phosphatidylcholin-Spiegels bei Kachexie-Patienten.

Ausgewählt für diese Studie wurden 30 Patienten mit unterschiedlichen soliden Tumoren. Die Patienten hatten entweder keinen Gewichtsverlust seit der Erstdiagnose (n: 11) oder aber einen Gewichtsverlust von < 10 % (n: 10) bzw. einen Gewichtsverlust von > 10 % (n: 9). Im Serum dieser Patienten wurde die Konzentration von Lyso-Phosphatidylcholin (Lyso-PC) mit Hilfe der HPTLC

(hochauflösende Dünnschichtchromatograpie) bestimmt. Hierzu wurden die Seren (500 µl) mit 500 µl 0,9%iger Kochsalzlösung verdünnt und 3 mal mit je 2ml Chloroform/Methanol (2:1, v/v) extrahiert. Die vereinigten organischen Phasen wurden mit Hilfe eines Stickstoffstromes zur Trockene eingedampft, und der Rückstand in je 300 µl Choroform/Methanol (2:1, v:v) aufgenommen. 50 µl dieser Lösungen wurden mit Hilfe eines Camag Auftrageautomaten auf Silicagel-Platten (für die HPTLC) aufgetragen (Strichlänge 5 mm), ebenfalls aufgetragen wurden entsprechende Lösungen von Lyso-Phosphatidylcholin (E. Merck, Darmstadt) in Chloroform/Methanol (2: 1) als Standards. Die Platten wurden in Choroform/Methanol/25%Ammoniak/Wasser (45/30/15/10) entwickelt und anschließend bei 180 °C getrocknet. Die Platten wurden in eine CuSO₄/Phosphorsäure-Lösung getaucht, getrocknet (bei ca. 60 °C) und bei 180 °C gefärbt. Die Intensität der Lyso-PC-Spots wurde mit Hilfe eines Camag-Scanners III quantifiziert und die Konzentration des Lyso-PC's in den Patienten-Seren mit Hilfe der mitaufgetragenen Standards berechnet. Fig. 4 zeigt das Ergebnis der Quantifizierung.

### Beispiel 5: MPL-indizierte Verringerung des Gewichtsverlustes von immunkompetenten Mäusen, die ein Nierenzellkarzinom (RENCA-Modell, orthotopes Modell) tragen.

Für dieses Experiment wurden weibliche BALB/c-Mäuse eingesetzt (Charles River, Frederick, Md.) in einem Alter von 6-8 Wochen (Gewicht: ca. 20 g). Tumorzellen (RENCA, murines Nierenzellkarzinom) wurden bei den Tieren während einer Operation durch Injektion in die Niere eingebraucht (Injektion in den subcapsularen Spalt in der linken Niere). Eine Woche nach Instillation von 10⁶ RENCA-Tumorzellen wurde mit der Behandlung der Mäuse begonnen (Tag 1). Zu diesem Zeitpunkt hatten die Mäuse ein Gewicht von 19-24 g. Die Tiere erhielten Wasser und Nagerfutter (Altromin, Lage, Deutschland) *ad libitum.* Der RENCA-Tumor bewirkt einen starken Gewichtsverlust (kachexieinduzierend).

Zur Therapie wurden marine Phospholipide (MPL) der Fa. BioSea, Norwegen, eingesetzt. Die Zusammensetzung dieser MPL war wie folgt:

### Gesamt-Zusammensetzung

| | |
|---|---|
| Gesamt-Lipide | 99,37 g/100 g |
| Wasser | 0,63 g/100 g |
| Neutrale Lipide | 67,72 g/100 g |
| Triglyceride | 61,37 g/100 g |
| Diglyceride | 4,98 g/100 g |
| Monoglyceride | 0,99 g/100 g |
| Cholesterin | 5,04 g/100 g |
| Freie Fettsäuren | 3,53 g/100 g |
| Polare Lipide | 32,75 g/100 g |
| Phosphatidylcholin | 28,64 g/100 g |
| 2-Lysophosphatidylcholin | 0,38 g/100 g |
| Phosphatidylinositol | 0,72 g/100 g |
| Sphingosin | 1,12 g/100 g |
| Phosphatidylethanolamin | 1,89 g/100 g |

Die Fettsäurerestverteilung in diesem MPL auf die neutralen und polaren Lipide war wie folgt:

| | Neutral | Polar | |
|---|---|---|---|
| Gesättigt | 15,6 | 22,8 | g/100 g Lipid |
| Einfach ungesättigt | 29,2 | 17,6 | g/100 g Lipid |
| Diene | 2,5 | 1,3 | g/100 g Lipid |
| Polyene | 52,6 | 58,3 | g/100 g Lipid |
| 20:5 | 18,8 | 16,5 | g/100 g Lipid |
| 22:6 | 22,8 | 33,7 | g/100 g Lipid |
| ? -3 / ? -6 | 12,4 | 21,0 | g/g |

Die marinen Phospholipide wurden in physiologischer Kochsalzlösung durch Ultraschall bei Raumtemperatur dispergiert. Es entstand eine heterogene Dispersion, die mit Ringerlösung auf 15 mg MPL/ml eingestellt wurde. Den Tieren wurden 10 rül/kg (d.h. 150 mg/kg) per Schlundsonde p.o. pro Tag an den Tagen 1-5, 8-12 und 15-20 zugeführt. Die Gewichte der Tiere wurden am letzten Tag verglichen. Die Gewichte wurden um die Masse des Tumors korrigiert.

Ergebnisse: Durch orale Gabe von MPL, 150 mg/kg/Tag, als Dispersion in Ringerlösung, konnte der Gewichtsverlust im Vergleich zu einer unbehandelten Kontrollgruppe signifikant vermindert werden (p: >0,002; Gruppe 1: 150 mg/kg/Tag MPL (n=5), Gewicht: 84,98 +/- 3,67 % des Ausgangsgewichtes, Gruppe 2: unbehandelte Kontrolle (n=5), Gewicht: 75,66 +/- 3,20 % des Ausgangsgewichtes).

### Beispiel 6: Wirksamkeit von MPL auf den Gewichtsverlauf von Nacktmäusen, die ein humanes Prostatakarzinom (LNCaP) tragen.

Für diese Experimente wurden männliche SCID-Mäuse (C.B-17/IcrHsd-Prkdcscid) (Harlan-Winkelmann, Borchen, Deutschland) in einem Alter von 6 bis 12 Wochen verwendet. Die Tiere hatten ein Gewicht von 25 bis 35 g und wurden unter einem natürlichen Tag/Nacht-Zyklus gehalten. Die Tiere erhielten Wasser und Nagerfutter (Altromin, Lage, Deutschland) *ad libitum.* Den Tieren wurden 2x10⁶ LNCaP-Zellen in 50 µl DMEM/Matrigel (1:1) subkutan in die rechte oder linke Flanke appliziert.

Die Therapieexperimente wurden begonnen, wenn die Tumore eine Größe von 30-40 mm³ erreicht hatten. Der Tumor wirkt kachexieinduzierend, wozu er allerdings die o.g. Größe erreicht haben muß.

Zur Therapie des Gewichtsverlustes wurde MPL der Fa. BioSea, 150 mg/ml eingesetzt (MPL wurde dazu in Kochsalzlösung (0,9%) durch Ultraschall dispergiert), so dass eine heterogene liposomale Dispersion entstand. Für die orale (p.o.) Applikation wurde die Dispersion mit Kochsalzlösung auf 15 mg/ml eingestellt.

Die Tiere wurden bis auf die Wochenden (5 Tage, dann 2 Tage Pause) über 5 Wochen mit 150 mg/kg MPL p.o. per Schlundsonde behandelt (Applikations-. volumen: 10 ml/kg). Kontrolltiere erhielten nur Kochsalzlösung.

Ergebnis: Durch orale Gabe von MPL als liposomale Dispersion in Kochsalzlösung konnte der Gewichtsverlust im Vergleich zur Kontrollgruppe vermindert werden. (Gruppe 1: 150 mg/kg/Tag MPL (n=8), Ausgangsgewicht: 25,00 ± 1,97 g, Endgewicht (35 d): 20,73 ± 1,62 (82,9 ± 6,8%); Gruppe 2: unbehandelte Kontrolle (n=3), Ausgangsgewicht: 31,98 ± 0,93 g; Endgewicht (35 d): 25,17 ± 1,06 (78,7 ± 3,3%).

### Beispiel 7: Palliativer Einsatz von hydrierten Phospholipiden an Patienten mit fortgeschrittenen Tumorerkrankungen; Gewichtsverlauf der Patienten.

An drei Patienten mit fortgeschrittener metastasierter Tumorerkrankung wurde ein Heilversuch mit hydrierten Phospholipiden durchgeführt. Die Patienten erhielten die Phospholipide (S-75-3N, Fa. Lipoid) wie folgt: in den ersten 4 Wochen 2 x 3 Tabletten (Tagesdosis 780 mg/Tag), ab der fünften Woche in Form von 3 x 3 Tabletten täglich (1170 mg/Tag) (je Tablette: 130 mg Phospholipid). S-75-3N ist hydriertes Phospholipid aus Soja mit einem Gehalt an Phosphatidylcholin und Lyso-Phosphatidylcholin von 81,7 % in der verwendeten Charge.

Die Patienten hatten unterschiedliche solide Tumoren:
Patient 1: 60 Jahre, männlich, hepatisch und pulmonal metastasiertes Rektumkarzinom, der Patient hatte zuvor bereits 8 verschiedene antitumorale Therapien erhalten;
Patient 2: 68 Jahre, weiblich, hepatisch, lymphogen und ossär metastasiertes Oropharynx-Karzinom, insgesamt 6 unterschiedliche antitumorale Vortherapien; Patient 3: 74 Jahre, männlich, unbekannter Primärtumor mit Peritonealkarzinose, insgesamt 5 antitumorale Vortherapien.
Einschlußkriterien: Einschlossen wurden Patienten, für die eine Therapie mit hydrierten Phospholipiden eine positiven Einfluß auf, die Lebensqualität haben konnte. Als Zielparameter wurden Schmerzen, Appetitverlust und Gewichtsverlust definiert.

Alle Patienten haben vor dem Beginn ihrer Behandlung mit hydriertem Phospholipid einen deutlichen Gewichtsverlust erlitten. Vor Behandlungsbeginn erhielten alle Patienten eine ausführliche Anamnese, körperliche Untersuchung sowie ein komplettes Labor (inklusive Lipidelektrophorese). Die notwendigen Staginguntersuchungen wurden baseline durchgeführt.

Nach jedem Einnahme-Zyklus von 4 Wochen wurden die Patienten eingehend untersucht (Verlaufskontrolle). Alle Patienten erhielten die Substanz als rein supportives Konzept, eine Kombination mit zytostatischer Therapie oder anderen antineoplastischen Substanzen war nicht vorgesehen. Es wurde darauf geachtet, dass ein Abstand von ca. 4 Wochen zwischen Ende der letzten antitumoralen Therapie und Beginn der Einnahme der Phospholipide bestand.

Die Daten der Patienten sind in der Tabelle 1 aufgeführt. Die Phospholipide wurden in allen Fällen gut vertragen und konnten von den Patienten kontinuierlich eingenommen werden. Es wurden bei keinem der Patienten Nebenwirkungen festgestellt. Es zeigte sich, dass der Gewichtsverlust nach Einnahme der Phospholipide stoppte bzw. sogar bei zwei Patienten eine Gewichtszunahme zu verzeichnen war (Patient 1 im zweiten Zyklus der Behandlung, Patient 2 im ersten Zyklus). Bei 2 Patienten verbesserte sich der initiale WHO-Status (Index für Lebensqualität) (Patient 1: WHO 1 --> WHO 0; Patient 3: WHO 2 --> 1), bei Patient 2 blieb er gleich (WHO 2). Infolge eines schweren respiratorischen Infekts verschlechterte sich der WHO bei Patient 1 zwischenzeitlich auf 1.

Bei keinem Patienten verstärkte sich durch die regelmäßige Einnahme der Phospholipide die Inappetenz (Anorexie). Es wurde auch keine Zunahme der Schmerzintensität im Behandlungszeitraum beobachtet. Bei Patient 1 war im Rahmen der ausgedehnten Lebermetastasierung der im Verhältnis dazu sehr geringe Schmerzmittelbedarf unter der Phospholipidtherapie besonders auffällig. Im 2. und 3. Behandlungszyklus (endend mit Tag 56 und 84) fand sogar keine regelmäßige Schmerzmedikation mehr statt, es wurde nur bei Bedarf Novalgin verabreicht.

Zwei Patienten sind mittlerweile an ihrer schweren Tumorerkrankung verstorben (Patient 2 und 3). Patient 1 nimmt die Phospholipide weiterhin (Monat 5). Dieser Patient hat zwischenzeitlich aufgrund einer schweren Infektion wieder Gewicht verloren (siehe Fig. 5), ist aber nach antibiotischer Therpie der Infektion wieder in einem verbesserten Zustand und nimmt wieder zu.

Bei Patient 1 konnte klinisch und bildgebend über 5 Monate ein stabiler Verlauf der Größe des Primärtumörs dokumentiert werden.

**Tabelle 1: Patientendaten**

| **Pat.-Nr.** | | **1** | **2** | **3** |
|---|---|---|---|---|
| | | | | |
| Alter | | 60 | 68 | 74 |
| Geschlecht | | m | w | m |
| Primartumor | | Rektum-Ca | Oropharynx-Ca | CUP |
| Metastasen | | Leber, Lunge | Leber, LK, Knochen | Peritoneum |
| Anzahl Vortherapien | | 8 | 6 | 5 |
| Beqleittherapien | | UMK | UMK, Navelbine oral | UMK |
| Start | | 13.12.2005 | 19.12.2005 | 05.01.2006 |
| verstorben | | nein | 11.3.2006 | 26.2.2006 |
| **WHO Baseline** (Tag 0) | | 1 | 2 | 2 |
| WHO Tag 28 | | 1 | 2 | 1 |
| WHO Tag 56 | | 0 | 2 | |
| WHO Tag 84 | | 0 | | |
| WHO Tag 114 | | 1 * | | |
| WHO Tag 128 | | 1 | | |
| WHO Tag 176 | | 1 | | |
| *Gewicht -6 Monate* | -180 | 93 | 59 | 77 |
| *Gewicht -3 Monate* | -90 | 88 | 60,5 | 67 |
| **Gewicht Baseline** | 0 | 85 | 55 | 60 |
| Gewicht Tag 28 | | 85 | 55 | 62,5 |
| Gewicht Tag 56 | | 90 | 53 | |
| Gewicht Tag 84 | | 88 | | |
| Gewicht Tag 114 | | 85 * | | |
| Gewicht Tag 128 | | 86 | | |
| Gewicht Tag 176 | | 87 | | |
| * schwerer Infekt (Bronchitis) seit 24.3.06; Gabe von Ciprobay und Prednison | | | | |

### Beispiel 8: Antimetastatsiche Wirksamkeit von hydrierten Phospholipiden.

Für dieses Experiment wurden weibliche thymusaplasierte Nacktmäuse (Hsd:Athymic Nude-Foxnlnu; Harlan-Winkelmann, Borchen, Deutschland) in einem Alter von 8-10 Wochen eingesetzt. Die Tiere hatten ein Gewicht von ca. 25 g und wurden unter einem natürlichen Tag/Nacht-Zyklus gehalten. Die Tiere erhielten Wasser und Nagerfutter (Altromin, Lage, Deutschland) *ad libitum.*

1 mm³ große Stücke des Pankreaskarzinoms MiaPaCa-2 (retroviral transfiziert mit dem Luciferase-Gen), wurden in den Pankreas der Mäuse orthotop implantiert. Nach 14 Tagen wurden die Tiere randomisiert (über die Luciferase-Lichtreaktion nach Injektion des Luciferase-Substrates Luciferin).

Hydriertes Ei-Lecithin (Ei-Phosphatidylcholin, Ei-PC; EPC-3, Fa. Lipoid, Ludwigshafen) wurde als liposomale Formulierung mit Cholesterol (Merck, Darmstadt) in einer Dosis von 0,454 mg EPC-3/kg Maus wöchentlich über 5 Wochen durch Injektion in die Schwanzvene von 10 Tieren gegeben (Tage: 0, 7, 14, 21, 28). Das molare Verhältnis zwischen dem Lecithin und Cholesterol betrug 55/45. Das Injektionsvolumen betrug 10 ml/kg. Kontrolltiere (n: 10) erhielten an den Tagen 0, 7, 14, 21, 28 nur Kochsalzlösung. Die Größe der Liposomen betrug im Mittel 36 nm.

Die Metastasierung in den Organen wurde durch Bestimmung der luciferase-Aktivität im Zellhomogenisat bestimmt und mit denen der Kontrolltiere verglichen.

Ergebnis: Die Gäbe von hydriertem Phospholipid in einer Dosis von 0,454 mg/kg/Woche i.v. bewirkte keine toxische Symptomatik bei den Tieren (Gewichtsverläufe identisch mit denen der Kontrolltiere). Bei den Tieren, die mit hydriertem Phospholipid behandelt wurden, zeigten sich weniger Tumorzellen in den Lebern, dem Hauptort der Metastasierung dieses Tumors, wodurch die Verminderung der Metastasierung durch die Phospholipidgabe gezeigt werden konnte (RLU: Kontrollen: 89,2 +/- 158,61; Behandelte Tiere: 11,2 +/- 10,9; vgl. Tabelle 2). Bei den behandelten Tieren hatten drei Tiere überhaupt keine Lebermetastasen, während in der Kontrollgruppe lediglich ein metastasenfreies Tier war.

**Tabelle 2: RLU der Lebern der Kontrolltiere und der mit hydriertem Phospholipid behandelten Tiere.**

| Kontrolltiere | Behandelte Tiere |
|---|---|
| Leber(RLU) | Leber(RLU) |
| 208,74 | 16,93 |
| 37,11 | 0,00 |
| 502,88 | 12,21 |
| 0,00 | 0,00 |
| 7,92 | 8,98 |
| 19,21 | 35,71 |
| 11,29 | 11,71 |
| 9,81 | 0,00 |
| 86,58 | 14,57 |
| 8,88 | 12,31 |

### Beispiel 9: Zusammensetzung der hydrierten Phospholipiden, die in den Beispielen eingesetzt wurden (S75-3, Lyso-PC und EPC3), und gaschromatographische Bestimmung ihrer Fettsäurerestzusammensetzung.

Die Hersteller der hier verwendeten hydrierten PL EPC-3 und S75-3-N geben deren Zusammensetzung wie folgt an:
Zusammensetzung von EPC-3, Charge 276015-1:

| Phosphatidylcholin | Spezifikation min. 98,0 % in Trockenmasse | Ergebnis 98,4 % |
|---|---|---|
| Phosphatidylethanolcholin | max. 0,1 % | < 0,1 % |
| Lysophosphatidylcholin | max. 0,5 % | < 0,1 % |
| Sphingomyelin | max. 1,0 % | < 0,1 % |
| Triglyceride | max. 0,5 % | < 0,2 % |
| Cholesterin | max. 0,5 % | < 0,1 % |
| Freie Fettsäuren | max. 0,2 % | < 0,05 % |
| D, 1-α-Tocopherol | max.0,1 % | < 0,1 % |
| Phosphor | 3,8 - 4,0 % | 3,88 % |
| Wasser (KF) | max. 2,0 % | 1,0 |
| Ethanol | max. 0,5 % | < 0,05 % |
| Peroxidzahl | max. 3 | 0,1 |
| Jodzahl | max. 3 | 0 |
| Restproteine | max. 50 ppm | < 50 ppm |
| Schwermetalle | max. 10 ppm | < 10 ppm |
| Endotoxine (LAL-Test) | max. 6 EU/g | < 1,2 EU/g |

Zusammensetzung von S75-3N, Charge 290077-1:

| | Spezifikation | Ergebnis | Methode |
|---|---|---|---|
| Phosphatidylcholin + Lysophosphatidylcholin | min. 70,0 % | 81,7 % | PC2 |
| Phosphor | 3,5 - 4,0 % | 3,7 % | TP |
| Wasser (KF) | max. 2,0 % | 0,7 % | USP <921> |
| Ethanol | max. 1,0 % | 0,1 % | ET |
| Jodzahl | max. 3,0 | 3,0 | JZ |

EPC-3 besteht also zu mindestens 98 % aus PC, S75-3N zu mindestens 70 % aus PC und Lyso-PC. Es sei angemerkt, dass in S75-3N keine weiteren Phospholipide enthalten sind.

Die Fettsäurerestzusammensetzung wurde gaschromatographisch wie folgt ermittelt:
Umesterung: Bei der Gaschromatographie ist die Flüchtigkeit der Analyten Grundvoraussetzung für ihre Bestimmung. Die Analyse von Fettsäuren mit Hilfe der Gaschromatographie (GC) erfordert daher zunächst eine Derivatisierung, um sie leicht verdampfbar zu machen. Im vorliegenden Fall wurden die Fettsäuren vor Analyse methyliert, die eigentlichen Analyten sind hier also nicht die derivatisierten Phospholipide, sondern die Methylester der in den Phospholipiden gebundenen Fettsäuren. Bei der im Folgenden beschriebenen Methylierung werden die an die Phospholipide gebundenen Fettsäuren zunächst vom Glyceringerüst abgespalten, dann methyliert (Umesterung).

Je etwa 1 mg der zu analysierenden Phospholipide wurden genau eingewogen und in jeweils 1 ml Hexan (Merck, Darmstadt, Deutschland) gelöst. Die Methylierung erfolgte mit Methanol mit Zusatz von Bortrifluorid, welches als fertiges Reagenz erhältlich ist (boron trifluoride methanol solution, 14%, von Sigma, Steinheim, Deutschland). Nach Zugabe von 1 ml des Reagenz wurde das Gemisch für 1 Stunde bei 100°C (Heizblock) in einem dicht verschlossenen Glasröhrchen erhitzt. Nach Abkühlen wurde 1 ml destilliertes Wasser hinzugegeben und 5 min bei 1000 rpm gevortext. Es folgte Zentrifugation für 5 min bei 3000 rpm. Die obere Hexanphase wurde mit einer Glaspasteurpipette abgenommen und in ein trockenes, sauberes Glasröhrchen überführt. Die abgetrennte Hexanphase wurde bei 40°C im Heizblock unter einem konstanten Stickstoffstrom abgedampft. Der trockene Rückstand wurde in 100 µl Hexan aufgenommen und wieder 5 min bei 1000 rpm gevortext. Die erhaltene Lösung wurde in ein GC-Autosampler Vial mit Insert überführt und mit einer Bördelkappe verschlossen. Anschließend wurde die GC durchgeführt (Gerät: HP 5890 Series II plus Gas Chromatograph).

Analysenbedingungen:
- Polar beschichtete Kapillarsäule (Länge 50 m, ID 0,32 mm)
- Trägergas Helium (Vordruck 170 kPa)
- FID-Detektor (Brenngase Wasserstoff und synthetische Luft, Makeup-Gas Helium)
- Gasfluss konstant 1 ml/min
- Splitverhältnis 1:100
- Injektortemperatur 260°C, Detektortemperatur 280°C
- Ofentemperaturprogramm: Initial 120°C für 1 min, 30°/min bis 200°, 10°/min bis 230°C, 230° für weitere 25 min
- Injektionsvolumen 1 µl

Auswertung: Die Interpretation des Chromatogramms geschieht mit Hilfe eines Standard-Gemisches aus 9 fertigen Fettsäure-Methylestern: C12:0, C14:0, C17:0, C16:0, C18:0, C18:1, C18:2, C18:3 und C20:0 (alle von Sigma, Steinheim, Deutschland; die Zahl nach dem Doppelpunkt gibt die Zahl der enthaltenen Doppelbindungen an), gelöst in Hexan.

Ergebnis: Dominierend sind die Peaks für die Palmitinsäuremethylester und die Stearinsäuremethylester. Die relative Fettsäurerestzusammensetzung der eingesetzten Phospholipide ist in Tabelle 3 dargestellt:

**Tabelle 3: Fettsäurerestzusammensetzung der eingesetzten PL in [%]**

| | S 75-3 | EPC3 | Lyso-PC |
|---|---|---|---|
| Behensäure (Docosansäure) | - | 3,1 | - |
| Arachinsäure (Eicosansäure) | - | 4,2 | - |
| Stearinsäure | 80,0 | 61,0 | 10,0 |
| Palmitinsäure | 18,6 | 30,9 | 90,0 |
| Myristinsäure | <0,5 | <0,5 | - |
| Laurinsäure | <0,5 | <0,5 | - |

Fettsäuren mit einer Kettenlänge unter 16 C-Atomen waren also nur zu weniger als 1 % aller Fettsäuren nachweisbar. Dagegen machten Stearinsäure und Palmitinsäure in allen verwendeten PL den Hauptanteil der Fettsäurereste aus.

### Beispiel 10: Aufnahme von Lyso-PC in Tumorzellen und Wirkung von Lyso-PC auf diese Zellen.

Zellen: Für dieses Experiment wurden folgende Prostatakarzinom-Zelllinien eingesetzt: DU145, PC-3, LNCaP.

Für die Messung des Lyso-PC-Verbrauchs einerseits und die Messung der Zellproliferation andererseits wurden unterschiedliche Kulturbedingungen gewählt:
Zellkultur und Inkubation mit Lyso-PC; Lyso-PC Verbrauch: 500 µl einer Zellsuspension mit einer Konzentration von 2x10⁵ Zellen/ml wurden in die Wells einer 24-well Zellkulturplatte ausgesät und bei 37°C und 5% CO₂ über Nacht inkubiert (DMEM (Dulbecco's Modified Eagle Medium; Gibco Invitrogen, Eggenstein, Deutschland, Best. Nr. 61965-026) plus 10% Fötales Kälberserum (Gibco Invitrogen)). In jedes Weil wurden anschließend 500 µl einer Lösung von 40 mg/ml Albumin (BSA; bovines Serumalbumin, Sigma, Taufkirchen, Best.-Nr. A9418-100) und 900 µM Lyso-Phosphatidylcholin (Lyso-PC, Sigma, Taufkirchen, Deutschland, Best.-Nr. L5254, Zusammensetzung siehe Beispiel 9) zugegeben (Endkonzentration im Weil: 20 mg/ml Albumin und 450 µM Lyso-PC). Die Zellen wurden für weitere 0, 6, 24, 48, 72 oder 144 h inkubiert.
Zellkultur und Inkubation mit Lyso-PC; Zellproliferation: 100 µl einer Zellsuspension mit einer Konzentration von 2x10⁵ Zellen/ml wurden in die Wells einer 96-well Zellkulturplatte ausgesät und bei 37°C und 5% CO₂ über Nacht inkubiert (DMEM (Dulbecco's Modified Eagle Medium; Gibco Invitrogen, Eggenstein, Deutschland, Best. Nr. 61965-026) plus 10% Fötales Kälberserum (Gibco Invitrogen)). In jedes Well wurden anschließend 100 µl einer Lösung von 40 mg/ml Albumin (BSA; bovines Serumalbumin, Sigma, Taufkirchen, Deutschland, Best.-Nr. A9418-100) und 900 µM Lyso-Phosphatidylcholin (Lyso-PC, Sigma, Taufkirchen, Deutschland, Best.-Nr. L5254, Zusammensetzung siehe Beispiel 9) zugegeben (finale Konzentration im Weil: 20 mg/ml Albumin und 450 µM Lyso-PC). Die Zellen wurden für weitere 0, 6, 24, 48 oder 72 h inkubiert.
Verbrauch von Lvso-PC in den Zellkulturen: Nach einer Inkubationszeit von 0, 6, 24, 48, 72 oder 144 h wurden die Platten zentrifugiert, der Überstand entnommen und bei -20 °C bis zur Bestimmung der Phosphocholin-enthaltenden Phospholipide gelagert.

Die Phosphocholin-enthaltenden Phospholipide wurden mit einem kommerziellen Test, dem "Phospholipids B - enzymatic colorimetric method" der Fa. Wako Chemicals (Neuss, Germany) nach Herstellerangaben bestimmt. Dieser Assay ist ein Assay zu Bestimmung von Phosphatidylcholin enthaltenden Phospholipdien in Serum oder Plasma. Die entsprechenden Phospholipide (Phosphatidylcholin, lyso-Phosphatidylcholin und Sphingomyelin) werden durch Phospholipase D hydrolysiert und das freie Cholin anschließend durch eine Cholinoxidase in Betain überführt. Das bei dieser Oxidation freiwerdende Wasserstoffperoxid wird anschließend genutzt, um 4-Aminoantipyrin und Phenol zu einem farbigen Analyten zu koppeln (Absorption: λₘₐₓ = 505 nm).

In einer transparenten 96-Well-Platte wurde in jedes Well 50 µl des Zellkulturüberstandes und 200 µl der Reaktionslösung des Assays gegeben. Die Reaktionslösung wurde zuvor hergestellt durch Zugabe von 45 ml Puffer (50 mM Tris, 5 mg/dL Kalziumchlorid, 0,05 % Phenol) zum "Färbereagenz" (Enzymgehalt in 45 ml: Phospholipase D 20 U, Cholinoxidase 90 U, Peroxidase 240 U; 4-Aminoantipyrin 0,015 %). Die Platten wurden zentrifugiert (1200 rpm, 560 x g, 1 min, zur Entfernung von Blasen) und für 20 Minuten bei 37 °C inkubiert. Die Absorption wurde bei 490 nm bestimmt, der Gehalt an PC-haltigen Phospholipiden wurde durch Vergleich mit einer Eichkurve (aufgenommen mit Cholinchlorid als Referenzsubstanz) bestimmt.

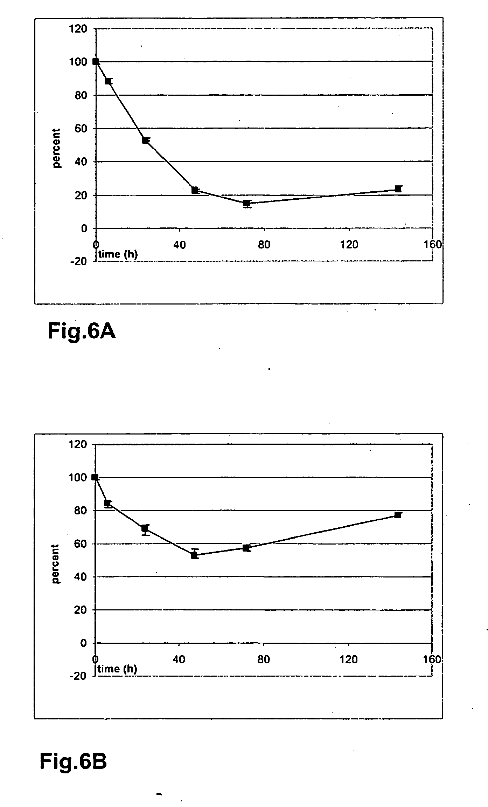

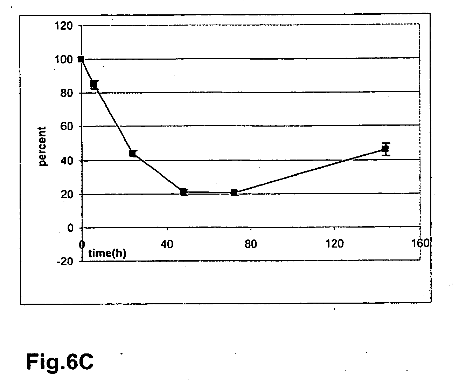

Ergebnisse: Die Abnahme der PC-haltigen Phosholipide im Überstand, welche wegen der initialen Zugabe von 450 µM Lyso-PC überwiegend Lyso-PC sind, ist für die Zellen in Fig. 6 dargestellt. Ca. 15 bis 20 % der Phospholipide im Überstand stammen aus den Zellkulturmedien..

Bestimmung der Zellproliferation mit Hilfe des BrdU-Assays (Roche): Der dem 3H-Thymindin-Assay ähnliche BrdU-Assay wurde gemäß der Herstelleranweisung durchgeführt: 100 µl Zellkulturmedium wurden aus jedem der Wells entfernt und 10 µl "BrdU-Labeling-Solution" zugegeben und für weitere vier Stunden inkubiert. Während dieser Zeit wird das Pyrimidin-Analoge 5-Brom-2'-deoxyuridin (BrdU) anstelle von Thymidin in die bei der Zellteilung neu entstehende DNA eingebaut. Die Kulturplatten wurden anschließend zentrifugiert (Raumtemperatur (RT), 1250 rpm, 560 x g, 5 min) und das Zellkulturmedium abgeklopft. Die Platte wurde anschließend eine Stunde bei 60°C getrocknet, und die DNA danach mit je 100 µl "FixDenat Solution" pro well denaturiert, so daß das BrdU durch einen Antikörper erkannt werden kann. Die Lösung wurde wiederum durch Abklopfen entfernt, 50 µl der "Anti-BrdU-POD Working Solution" zugegeben und die Platten für 90 min bei RT inkubiert. Der enthaltene Antikörper-Peroxidase-Komplex bindet an das in der neuen DNA eingebaute BrdU. Nicht gebundenes BrdU-POD wird wiederum durch Abklopfen entfernt und die Wells werden einmal 200 µl und dann zweimal mit 100 µl "Washing Solution" gewaschen. Überschüssige Flüssigkeit wird durch Abklopfen entfernt. Anschließend werden 100 µl / Well "Substrate Solution" zugegeben und es wird für 20 Minuten bei RT inkubiert. Die Peroxidasereaktion wird durch Zugabe von 25 µl 1 N H₂SO₄ gestoppt, die Platten für eine Minute geschüttelt (300 rpm) und die Absorption in einem ELISA-Reader bei 450 nm gemessen. Die Intensität der Färbung korreliert direkt mit der DNA-Synthese und ist ein Maß für die Zahl der proliferienden Zellen in der Kultur. Je intensiver die Färbung bzw. OD bei 450 nm, desto mehr BrdU wurde eingebaut und desto stärker war somit die Proliferation der Zellen. Ergebnisse siehe Tabelle 4.

**Tabelle 4: Ergebnisse Proliferationsmessung. Der obenstehende Wert in jeder Zelle ist die OD der Zellen ohne Lyso-PC-Zusatz, der untenstehende Wert die OD der Zellen mit Lyso-PC-Zusatz.**

| Zeit [h] | LNCaP | PC3 | DU145 |
|---|---|---|---|
| 0 | 0,55+-0,05 | 1,11+-0,09 | 0,31+-0,04 |
| | 0,60+-0,02 | 1,16+-0,06 | 0,28+-0,02 |
| 24 | 1, 30+-0, 24 | 1,64+-0,13 | 0, 29+-0, 03 |
| | 1,08+-0,04 | 2,25+-0,17 | 0,22+-0,02 |
| 48 | 0,95+-0,14 | 1,64+-0,11 | 0,31+-0,03 |
| | 0,93+-0,05 | 1,57+-0,16 | 0,29+-0,02 |
| 72 | 1,31+-0,08 | 1,33+-0,10 | 0,39+-0,04 |
| | 1,09+-0,06 | 1,56+-0,11 | 0,30+-0,02 |

Trotz des Zusatzes von 450 µM Lyso-PC zu den Zellkulturen und der beobachteten Aufnahme des Lyso-PC durch die Zellen konnte keine Steigerung oder Abschwächung des Wachstums der Zelllinien PC-3, DU145 und LNCaP gesehen werden. Die gemessenen Unterschiede liegen im Bereich der üblichen Schwankungen in biologischen Systemen.

### Beispiel 11: Wirkung von EPC-3 (hydriertes Ei-Lecition auf die Proliferation von Prostata-Karzinomzellen.

Zellen: Für dieses Experiment wurden folgende Prostatakarzinom-Zelllinien eingesetzt: DU145, PC-3, LNCaP.

100 µl einer Zellsuspension mit einer Konzentration von 2x10⁵ Zellen/ml wurden in die Wells einer 96-well Zellkulturplatte ausgesät und bei 37°C und 5% CO₂ über Nacht inkubiert (DMEM (Dulbecco's Modified Eagle Medium; Gibco Invitrogen, Eggenstein, Deutschland, Best. Nr. 61965-026) plus 10% Fötales Kälberserum (Gibco Invitrogen)). In jedes Weil wurden anschließend 100 µl einer Liposomen-Präparation aus 45 mol% Cholesterol und 55 mol% EPC-3 (hydriertes PC aus Ei, Fa. Lipoid, Ch. Nr. 276015-1; Zusammensetzung siehe Beispiel 9 und xx) gegeben (Endkonzentration des EPC-3 in den Liposomen: 55 mol%). Die Liposomen-Präparation wurde mit Hilfe einer dualen asymmetrischen Zentrifuge (DAZ) hergestellt wie in WO 2006/069985 (dort Beispiel 1) beschrieben. Der mittlere Durchmesser der Liposomen betrug 37 nm. Die Endkonzentration EPC-3 in den Wells betrug 24,1 µM. In die Kontrollen wurde das gleiche Volumen (100 µl) 0,9%iger Kochsalzlösung gegeben. Nach 48 und 72 h Inkubation wurde ein BrdU-Zellproliferationsassay durchgeführt, wie er in Beispiel 10 beschrieben ist.

Ergebnis: 24,1 µm hydriertes Phosphatidylcholin hatte bei allen drei untersuchten Prostatazelllinien keinen Effekt auf die Proliferation. Die Ergebnisse (OD-Werte) sind in Tabelle 5 dargestellt. Die Werte der einzelnen Zelllinien zu den jeweiligen Zeitpunkten sind im Rahmen der üblichen Schwankungen biologischer Systeme als gleich anzusehen. EPC-3 hatte also keinen Einfluss auf die Zellproliferation.

**Tabelle 5: Darstellung des Einflusses von 24,1 µM hydriertem Phosphatidylcholin auf die Proliferation von drei verschiedenen Prostatakarzinomzelllinien (LNCaP, PC3, DU145). Es werden die OD-Werte aus einem BrdU-Assay präsentiert.**

| Zeit | LNCaP | | PC3 | | D145 | |
|---|---|---|---|---|---|---|
| | Kontrolle | EPC3 | Kontrolle | EPC3 | Kontrolle | EPC3 |
| 48 h | 1,00+- | 0,88+- | 0,86+- | 0,78+- | 1,43+- | 1,49+- |
| | 0,03 | 0,10 | 0,09 | 0,18 | 0,02 | 0,03 |
| 72 h | 1,14+- | 1,17+- | 0,78+- | 0,80+- | 1,46+- | 1,70+- |
| | 0,19 | 0,04 | 0,13 | 0,06 | 0,10 | 0,24 |

## Patentansprüche

1. Verwendung von einem oder mehreren Acylglycerophospholipiden (AGPL) mit einer Struktur der Formel (I) 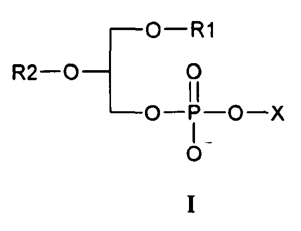 worin
R¹ und R² unabhängig voneinander ausgewählt sind aus H und C₁₆₋₂₄ Acylresten, die geradkettig, verzweigt oder zyklisch, gesättigt oder ungesättigt und mit 1 bis 3 Resten R³ substituiert sein können und in denen eines oder mehrere der C-Atome durch O oder NR⁴ ersetzt sein können;
X ausgewählt ist aus H, -(CH₂)ₙ-N(R⁴)₃⁺, -(CH₂)ₙ-CH(N(R⁴)₃⁺)-COO- und -(CH2)ₙ-CH(OH)-CH₂OH, wobei n eine ganze Zahl von 1 bis 5 ist;
R³ unabhängig vom Auftreten weiterer R³-Reste ausgewählt ist aus H, Niederalkyl, F, Cl, CN und OH; und
R⁴ unabhängig vom Auftreten weiterer R⁴-Reste ausgewählt ist aus H, CH₃ und CH₂CH₃,
oder ein pharmakologisch geeignetes Salz desselben als Wirkstoff zur Herstellung eines Medikaments zur Therapie oder Prophylaxe von Krebs-Begleiterscheinungen, ausgewählt aus Tumorkachexie, tumorinduzierten Schmerzzuständen und tumorinduzierter Fatigue.

2. Verwendung nach Anspruch 1, wobei die AGPL ausgewählt sind aus 1,2-Diacylglycerophospholipiden, 1-Acylglycerophospholipiden und 2-Acylglycerophospholipiden mit gesättigten oder ungesättigten Acylresten oder deren pharmazeutisch geeigneten Salzen, und bevorzugt Phosphatidylcholine sind.

3. Verwendung nach Anspruch 1 oder 2, wobei die AGPL Acylreste enthalten, die ausgewählt sind aus der Gruppe bestehend aus gesättigten Acylresten, Omega-3- und Omega-9-Fettsäureresten sowie Mischungen derselben, und der Gehalt der Acylreste aus dieser Gruppe in den AGPL bevorzugt mindestens 50 % aller in den AGPL enthaltenen Acylreste beträgt.

4. Verwendung nach einem oder mehreren der Ansprüche 1 bis 3, wobei die Acylreste geradkettig und unverzweigt sind.

5. Verwendung nach einem oder mehreren der Ansprüche 1 bis 4, wobei die Acylreste gesättigte Acylreste sind und die AGPL bevorzugt lediglich hydrierte oder gesättigte Acylreste enthalten.

6. Verwendung nach Anspruch 5, wobei die AGPL Phosphatidylcholine mit gesättigten Acylresten sind, bevorzugt Dipalmitoylphosphatitylcholin (DPPC) oder hydriertes Ei- oder Sojalecithin ist.

7. Verwendung nach einem oder mehreren der Ansprüche 1 bis 6, wobei die AGPL in Form von Lecithin, bevorzugt von hydriertem Lecithin, vorliegen.

8. Verwendung nach einem oder mehreren der Ansprüche 5 bis 7, wobei der Anteil der AGPL an den Gesamtlipiden im Medikament wenigstens 10 Gew.-%, bevorzugt wenigstens 40 Gew.-%, ganz besonders bevorzugt 100 Gew.-% beträgt.

9. Verwendung nach einem oder mehreren der Ansprüche 1 bis 4, wobei die Acylreste Omega-3- und/oder Omega-9-Fettsäurereste sind, bevorzugt Omega-3-Fettsäurereste sind.

10. Verwendung nach Anspruch 9, wobei die AGPL
(i) überwiegend langkettige Omega-3-Fettsäurereste tragen (Omega-3-Fettsäurerest-reiche AGPL), bevorzugt mit Kettenlängen von mindestens C20, insbesondere Docosahexaensäurereste (DHA) und/oder Eicosapentensäurereste (EPA); oder
(ii) überwiegend Omega-9-Fettsäurereste tragen (Omega-9-Fettsäurerest-reiche AGPL), bevorzugt mit Kettenlängen von mindestens C18, insbesondere Ölsäurereste,
und wobei der Gehalt an Omega-3- bzw. Omega-9-Fettsäureresten bevorzugt wenigstens 20 %, besonders bevorzugt wenigstens 35 %, ganz besonders bevorzugt wenigstens 45 % aller in den AGPL enthaltenen Acylreste beträgt.

11. Verwendung nach Anspruch 9 oder 10, wobei das Gewichtsverhältnis von Omega-3-Fettsäureresten und/oder Omega-9-Fettsäureresten zu Omega-6-Fettsäureresten in den verwendeten AGPL wenigstens 10 : 1, bevorzugt wenigstens 15 : 1 beträgt.

12. Verwendung nach einem oder mehreren der Ansprüche 9 bis 11, wobei die AGPL
(i) synthetische AGPL sind;
(ii) marine AGPL sind und bevorzugt aus marinen Lipidquellen, besonders bevorzugt aus tierischen Wasserbewohnern wie Fisch, ganz besonders bevorzugt aus Fischleber oder Fischrogen stammen; oder
(iii) in Form von Lecithin vorliegen.

13. Verwendung nach einem oder mehreren der Ansprüche 9 bis 12, wobei die Omega-3- und/oder Omega-9-Fettsäurerest-reichen AGPL im Gemisch mit anderen Substanzen eingesetzt werden, bevorzugt im Gemisch mit Triglyceriden, und wobei bevorzugt der Anteil der AGPL wenigstens 5 Gew.-%, besonders bevorzugt wenigstens 15 Gew.-% der Gesamtlipide in der verwendeten Zusammensetzung beträgt.

14. Verwendung nach einem oder mehreren der Ansprüche 1 bis 13, wobei die AGPL eine Mischung aus gesättigten Acylresten, Omega-3- und Omega-9-Fettsäureresten enthalten.

15. Verwendung nach einem oder mehreren der Ansprüche 1 bis 14, wobei die AGPL der einzige pharmakologisch aktive Wirkstoff des Medikaments sind.

16. Verwendung nach einem oder mehreren der Ansprüche 1 bis 14, wobei das Medikament weiterhin als zusätzliche Bestandteile
(i) Triglyceride, freie Fettsäuren, Diglyceride und/oder Monoglyceride, wobei der Gesamtanteil der genannten Verbindungen vorzugsweise maximal 90 Gew.-% aller im Medikament vorhandene Lipide beträgt, und/oder
(ii) Substanzen mit Wirkung auf den Phospholipidmetabolismus, bevorzugt CyP450-Inhibitoren, Cyclooxygenase-Inhibitoren oder Phospholipase-A2-Inhibitoren,
enthält.

17. Verwendung nach Anspruch 16, wobei der zusätzliche Bestandteil des Medikaments ein oder mehrere Triglyceride sind, und wobei bevorzugt das Verhältnis AGPL : Triglycerid gleich oder größer 1 : 9 ist.

18. Verwendung nach einem oder mehreren der Ansprüche 1 bis 16, wobei das Medikament zur oralen oder intravenösen Gabe geeignet ist und bevorzugt zur oralen Verabreichung geeignet ist.

## Claims

1. Use of one or more acylglycerophospholipids (AGPLs) having a structure of formula (I) 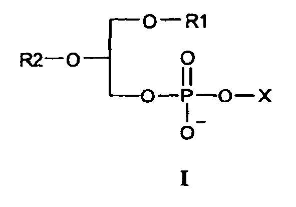 wherein
R¹ and R² are independently selected from H and C₁₆-₂₄ acyl residues, which may be linear, branched or cyclic, saturated or unsaturated, and may be substituted with 1 to 3 residues R³, and one or more of the carbon atoms may be replaced by O or NR⁴;
X is selected from H, -(CH₂)ₙ-N(R⁴)₃⁺, -(CH₂)ₙ-CH(N(R⁴)₃⁺)-COO- and -(CH₂)ₙ-CH(OH)-CH₂OH, wherein n is an integer of from 1 to 5;
R³ independently of the occurrence of further R³ residues is selected from H, lower alkyl, F, Cl, CN and OH; and
R⁴ independently of the occurrence of further R⁴ residues is selected from H, CH₃ and CH₂CH₃;
or of a pharmacologically acceptable salt thereof as an active substance for the preparation of a medicament for the therapy or prophylaxis of symptoms concomitant with cancer, selected from tumor cachexia, tumor-induced pain conditions and tumor-induced fatigue.

2. The use according to claim 1, wherein said AGPLs are selected from 1,2-diacylglycerophospholipids, 1-acylglycerophospholipids and 2-acylglycerophospholipids with saturated or unsaturated acyl residues or pharmaceutically acceptable salts thereof, and are preferably phosphatidylcholines.

3. The use according to claim 1 or 2, wherein said AGPLs contain acyl residues selected from the group consisting of saturated acyl residues, ω-3 and ω-9 fatty acid residues and mixtures thereof, and the content of acyl residues from such group in the AGPLs is preferably at least 50% of all acyl residues contained in the AGPLs.

4. The use according to one or more of claims 1 to 3, wherein said acyl residues are linear and not branched.

5. The use according to one or more of claims 1 to 4, wherein said acyl residues are saturated acyl residues and said AGPLs preferably contain only hydrogenated or saturated acyl residues.

6. The use according to claim 5, wherein said AGPLs are phosphatidylcholines with saturated acyl residues, preferably dipalmitoylphosphatidylcholine (DPPC) or hydrogenated egg or soybean lecithin.

7. The use according to one or more of claims 1 to 6, wherein said AGPLs are in the form of lecithin, preferably hydrogenated lecithin.

8. The use according to one or more of claims 5 to 7, wherein the proportion of AGPLs in the total lipids in the medicament is at least 10% by weight, preferably at least 40% by weight, more preferably 100% by weight.

9. The use according to one or more of claims 1 to 4, wherein said acyl residues are ω-3 and/or ω-9 fatty acid residues, preferably ω-3 fatty acid residues.

10. The use according to claim 9, wherein said AGPLs
(i) predominantly bear long-chain ω-3 fatty acid residues (AGPLs rich in ω-3 fatty acid residues), preferably having chain lengths of at least C20, especially docosahexaenoic acid (DHA) and/or eicosapentaenoic acid (EPA) residues; or
(ii) predominantly bear ω-9 fatty acid residues (AGPLs rich in ω-9 fatty acid residues), preferably having chain lengths of at least C18, especially oleic acid residues; and
wherein the content of ω-3 or ω-9 fatty acid residues is preferably at least 20%, more preferably at least 35%, even more preferably at least 45% of all acyl residues contained in the AGPLs.

11. The use according to claim 9 or 10, wherein the weight ratio of ω-3 fatty acid residues and/or ω-9 fatty acid residues to ω-6 fatty acid residues in the AGPLs employed is at least 10:1, preferably at least 15:1.

12. The use according to one or more of claims 9 to 11, wherein said AGPLs
(i) are synthetic AGPLs;
(ii) are marine AGPLs, preferably originating from marine lipid sources, more preferably from animals from aquatic habitats, such as fish, even more preferably from fish liver or fish roe; or
(iii) are present in the form of lecithin.

13. The use according to one or more of claims 9 to 12, wherein said AGPLs rich in ω-3 and/or ω-9 fatty acid residues are employed in admixture with other substances, preferably in admixture with triglycerides, and wherein the proportion of the AGPLs is at least 5% by weight, more preferably at least 15% by weight, of the total lipids in the composition employed.

14. The use according to one or more of claims 1 to 13, wherein said AGPLs contain a mixture of saturated acyl residues, ω-3 and ω-9 fatty acid residues.

15. The use according to one or more of claims 1 to 14, wherein said AGPLs are the only pharmacologically active substance of the medicament.

16. The use according to one or more of claims 1 to 14, wherein said medicament further contains as additional components:
(i) triglycerides, free fatty acids, diglycerides and/or monoglycerides, wherein the total proportion of the compounds mentioned is preferably at most 90% by weight of all lipids present in the medicament; and/or
(ii) substances having activity on the phospholipid metabolism, preferably CyP450 inhibitors, cyclooxygenase inhibitors or phospholipase A2 inhibitors.

17. The use according to claim 16, wherein said additional component of the medicament is one or more triglycerides, wherein preferably the ratio of AGPL to triglyceride is equal to or greater than 1:9.

18. The use according to one or more of claims 1 to 16, wherein said medicament is suitable for oral or intravenous administration, preferably for oral administration.

## Revendications

1. Utilisation d'un ou de plusieurs acylglycérophospholipides (AGPL) présentant une structure de formule (I) 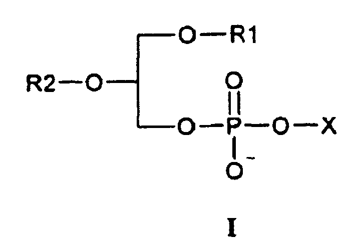 dans laquelle
R¹ et R² sont sélectionnés indépendamment l'un de l'autre parmi H et des radicaux acyle en C₁₆₋₂₄ qui sont linéaires, ramifiés ou cycliques, saturés ou insaturés, et peuvent être substitués par 1 à 3 radicaux R³, et dans lesquels un ou plusieurs des atomes de C peuvent être remplacés par O ou NR⁴ ;
X est sélectionné parmi H, -(CH₂)n-N(R⁴)₃⁺, -(CH₂)ₙ-CH(N(R⁴)₃⁺)-COO⁻ et -(CH₂)ₙ-CH(OH)-CH₂OH, où n est un nombre entier compris entre 1 et 5 ;
R³, indépendamment de la formation d'autres radicaux R³, est sélectionné parmi H, un alkyle inférieur, F, Cl, CN et OH ; et
R⁴, indépendamment de la formation d'autres radicaux R⁴, est sélectionné parmi H, CH₃ et CH₂CH₃,
ou d'un sel de celui-ci approprié sur le plan pharmacologique en tant que principe actif pour fabriquer un médicament destiné à la thérapie ou à la prophylaxie des effets secondaires du cancer, sélectionnés parmi une cachexie tumorale, des états douloureux induits par une tumeur ou une fatigue induite par une tumeur.

2. Utilisation selon la revendication 1, dans laquelle les AGPL sont sélectionnés parmi des 1,2-diacylglycérophospholipides, des 1-acylglycérophospholipides et des 2-acylglycérophospholipides présentant des radicaux acyle saturés ou insaturés, ou leurs sels appropriés sur le plan pharmaceutique, et sont de préférence des phosphatidylcholines.

3. Utilisation selon la revendication 1 ou 2, dans laquelle les AGPL contiennent des radicaux acyle qui sont sélectionnés dans le groupe composé de radicaux acyle saturés, d'esters d'acides gras oméga-3 et oméga-9 ainsi que de mélanges de ceux-ci, et dans laquelle la teneur en radicaux acyle issus de ce groupe dans les AGPL est de préférence au moins égale à 50 % de tous les radicaux acyle contenus dans les AGPL.

4. Utilisation selon une ou plusieurs des revendications 1 à 3, dans laquelle les radicaux acyle sont à chaîne linéaire et non ramifiés.

5. Utilisation selon une ou plusieurs des revendications 1 à 4, dans laquelle les radicaux acyle sont des radicaux acyle saturés et les AGPL contiennent de préférence uniquement des radicaux acyle hydrogénés ou saturés.

6. Utilisation selon la revendication 5, dans laquelle les AGPL sont des phosphatidylcholines présentant des radicaux acyle saturés, de préférence sont la dipalmitoylphosphatidylcholine (DPPC) ou la lécithine d'oeuf ou de soja hydrogénée.

7. Utilisation selon une ou plusieurs des revendications 1 à 6, dans laquelle les AGPL se présentent sous la forme de lécithine, de préférence de lécithine hydrogénée.

8. Utilisation selon une ou plusieurs des revendications 5 à 7, dans laquelle la fraction des AGPL par rapport au total des lipides dans le médicament est au moins égale à 10 % en poids, de préférence au moins égale à 40 % en poids, et est de façon particulièrement préférée égale à 100 % en poids.

9. Utilisation selon une ou plusieurs des revendications 1 à 4, dans laquelle les radicaux acyle sont des radicaux acides gras oméga-3 et/ou oméga-9, de préférence des radicaux acides gras oméga-3.

10. Utilisation selon la revendication 9, dans laquelle les AGPL
(i) portent essentiellement des radicaux acides gras oméga-3 à chaîne longue (AGPL enrichis en radicaux acides gras oméga-3), de préférence ayant des longueurs de chaîne d'au moins C20, en particulier les radicaux acide docosahexaénoïque (DHA) et/ou les radicaux acide éicosapentanoïque (EPA) ; ou
(ii) portent essentiellement des radicaux acides gras oméga 9 (AGPL enrichis en radicaux acides gras oméga 9), de préférence présentant des longueurs de chaîne d'au moins C18, en particulier des radicaux acide oléique,
et dans laquelle la teneur en radicaux acides gras oméga 3 ou oméga 9 est de préférence au moins égale à 20 %, de façon particulièrement préférée au moins égale à 35 %, et de façon tout particulièrement préférée au moins égale à 45 % de tous les radicaux acyle contenus dans les AGPL.

11. Utilisation selon la revendication 9 ou 10, dans laquelle le rapport pondéral entre les radicaux acides gras oméga 3 et/ou acides gras oméga 9 et les radicaux acides gras oméga 6 dans les AGPL utilisés est au moins égal à 10:1, de préférence au moins égal à 15:1.

12. Utilisation selon une ou plusieurs des revendications 9 à 11, dans laquelle les AGPL
(i) sont des AGPL synthétiques ;
(ii) sont des AGPL marins et proviennent de préférence de sources lipidiques marines, de façon particulièrement préférée d'animaux marins, comme le poisson, de façon tout particulièrement préférée de foie de poisson ou d'oeufs de poisson, ou
(iii) se présentent sous forme de lécithine.

13. Utilisation selon une ou plusieurs des revendications 9 à 12, dans laquelle les AGPL enrichis en radicaux acyle oméga 3 et/ou oméga 9 sont utilisés en mélange avec d'autres substances, de préférence en mélange avec des triglycérides, et dans laquelle de préférence la fraction des AGPL est au moins égale à 5 % en poids, de façon particulièrement préférée au moins égale à 15 % en poids du total des lipides de la composition utilisée.

14. Utilisation selon une ou plusieurs des revendications 1 à 13, dans laquelle les AGPL contiennent un mélange de radicaux acyle saturés, de radicaux acides gras oméga 3 et oméga 9.

15. Utilisation selon une ou plusieurs des revendications 1 à 14, dans laquelle les AGPL sont le seul principe actif du médicament actif sur le plan pharmacologique.

16. Utilisation selon une ou plusieurs des revendications 1 à 14, dans laquelle le médicament contient en outre en tant qu'ingrédients supplémentaires
(i) des triglycérides, des acides gras libres, des diglycérides et/ou des monoglycérides, où la fraction totale des composés nommés est de préférence au maximum égale à 90 % en poids de tous les lipides présents dans le médicament, et/ou
(ii) des substances ayant une action sur le métabolisme des phospholipides, de préférence des inhibiteurs de CyP450, des inhibiteurs de cyclooxygénase ou des inhibiteurs de phospholipase A2.

17. Utilisation selon la revendication 16, dans laquelle l'ingrédient supplémentaire du médicament est un ou plusieurs triglycérides, et dans laquelle de préférence, le rapport AGPL:triglycéride est supérieur ou égal à 1:9.

18. Utilisation selon une ou plusieurs des revendications 1 à 16, dans laquelle le médicament est approprié pour une administration par voie orale ou par voie intraveineuse, et est de préférence approprié pour une administration par voie orale.
